# FASCICULE DE BREVET EUROPEEN

(11) **EP 0 669 331 B1**
(45) Date de publication et mention de la délivrance du brevet: **11.10.2000**
(21) Numéro de dépôt: 95400382.8
(22) Date de dépôt: 23.02.1995
(51) Int. Cl.: C07D 405/12, C07D 319/18, C07D 319/20, A61K 31/36

(54) **Dérivés de benzodioxane, leur procédé de préparation et les compositions pharmaceutiques qui les contiennent**
Benzodioxan Derivate, Verfahren zu deren Herstellung und diese enthaltende pharmazeutische Zusammenstellungen
Benzodioxane derivatives, process for their preparation and pharmaceutical compositions containing them

(30) Priorité: 25.02.1994 FR 9402161
(43) Date de publication de la demande: 30.08.1995
(73) Titulaire: ADIR ET COMPAGNIE, 92415 Courbevoie Cédex (FR)
(72) Inventeur: Lavielle, Gilbert, F-78170 La Celle Saint Cloud (FR); Hautefaye, Patrick, F-77170 Servon Brie Comte Robert (FR); Muller, Olivier, F-95300 Ennery (FR); Millan, Mark, F-75017 Paris (FR); Brocco, Mauricette, F-75003 Paris (FR)

(56) Documents cités:
- EP-A- 0 389 352
- EP-A- 0 446 921
- FR-A- 1 343 644
- US-A- 3 225 052
- CHEMICAL ABSTRACTS, vol. 65, no. 68, 1966, Columbus, Ohio, US; abstract no. 1253b, G. POLUKORDAS ET AL. 'PHARMACOLOGICAL ACTIVITY OF SOME 5- AND 6-HYDROXY-1,4-BENZODIOXAN AMINOETHYL ETHERS.' & FARMAKOL. I TOKSIKOL., vol.28, no.5, 1965, RUSS. pages 603 - 607
- CHEMICAL ABSTRACTS, vol. 75, no. 28, 1971, Columbus, Ohio, US; abstract no. 5818f, V.DAUKSAS ET AL. 'IONISATIN CONSTANTS OF (OMEGA)AMINOALKYL ETHERS OF 5-HYDROXY-1,4-BENZODIOXANS' page 485 ; & LIET. TSR AUKST. MOKYKLU MOSKLO DARB.,CHEM. TECHNOL., vol.11, 1970, USSR pages 191 - 200
- CHEMICAL ABSTRACTS, vol. 72, no. 11, 1970, Columbus, Ohio, US; abstract no. 55353j, V. DAUKSAS ET AL. 'SYNTHESIS OF AMINOALKYL ETHERS OF 5-HYDROXY-1,4-BENZODIOXANES.' page 422 ; & LIET. TSR AUKST. MOKYKLU MOKSLO DARB., CHEM. TECHNOL., vol.9, 1968, USSR pages 101 - 107

## Description

La présente invention concerne de nouveaux dérivés de benzodioxane, leur procédé de préparation et les compositions pharmaceutiques qui les contiennent.

Un certain nombre de dérivés de 1,4-benzodioxane ont été décrits dans la littérature. C'est le cas plus particulièrement des composés décrits dans les brevets EP 307970, EP 210581 ou FR 2449088, qui présentent une activité vasodilatatrice ou bien des composés décrits dans le brevet EP 446921 qui possèdent des propriétés anxiolytiques.

US-A-3 225 052 (Exemple I) décrit le composé antipsychotique, Oxiperomide.

Les composés de la présente invention, outre le fait qu'ils soient nouveaux, présentent des propriétés pharmacologiques particulièrement intéressantes. En effet, un des défis de la psychopharmacologie aujourd'hui est de trouver de nouveaux médicaments permettant un meilleur contrôle de la schizophrénie dont le traitement est actuellement peu satisfaisant. Les neuroleptiques classiques, tel que l'halopéridol, soignent assez bien les symptômes productifs (tels que la fabulation et les hallucinations) mais leur efficacité vis-à-vis des symptômes déficitaires (tels que le retrait social) est très faible. De plus, ils provoquent un syndrome extrapyramidal de type Parkinsonien comme l'ont indiqué A.Y. DEUTCH et coll. (Schizophrenia Research, 4, 121-151, 1991) et H.Y. MELTZER et coll. (Pharmacol. Rev., 43, 587-604, 1991).
A la différence de l'halopéridol, la clozapine est plus efficace en traitant les symptômes déficitaires et elle est même efficace chez les patients qui résistent à l'halopéridol. De surcroit, elle ne provoque quasiment pas de syndrome extrapyramidal comme l'ont indiqué COWARD et coll. (Psychopharmacology, 99, s6-s12, 1989). Cette différence semble être due au profil réceptoriel de la clozapine qui est différent de celui de l'halopéridol: par exemple, sa faible activité relative sur les récepteurs D₂ et sa relativement forte affinité sur les récepteurs adrénergiques (α₁) et sérotinergiques (5-HT_{2A}, 5-HT_{2C}). Ces résultats ont été indiqués par H. CANTON et coll. (Eur. J. Pharmacol., 191, 93-96, 1990), ainsi que par A.Y. DEUTCH et H.Y. MELTZER cités plus haut.
De plus, à la différence de l'halopéridol, la clozapine montre une certaine affinité pour les récepteurs 5-HT_{1A}, dont l'activation est associée aux effets anxiolytiques, antidépresseurs et, éventuellement même, antipsychotiques comme l'ont indiqué S. AHLENIUS (Pharmacol. & Toxicol., 64, 3-5, 1989), J. E. BARRETT et coll. (in "5-HT_{1A} agonists, 5-HT₃ antagonists and benzodiazepines : their comparative behavioural pharmacology", Ed. R.J. ROGERS and S.J. COOPER, WILEY & Sons Ltd, Chichester, p. 59-105, 1991), M.J. MILLAN et coll. (Drug News Perspectives, 5, 397-466, 1992), J.M.A. SITSEN (DRUG NEWS & Perspectives, 4,414-418, 1991).

Néanmoins, en raison de sa toxicité, la clopazine ne peut pas être envisagée dans le traitement général des états schizophréniques. Il était donc particulièrement intéressant de trouver des produits partageant le mécanisme de cette dernière mais dépourvus de ses effets toxiques qui sont directement reliés à sa structure chimique particulière.

Les composés décrits dans ce brevet correspondent au profil recherché. En effet, ils présentent (in vitro et in vivo) un profil biochimique et fonctionnel très similaire à celui de la clozapine et avec de surcroit des affinités pour les récepteurs 5-HT_{1A} plus importantes. Ils possèdent donc un profil original qui semble être bien adapté à un meilleur traitement de la schizophrénie en comparaison de ce qu'il est actuellement possible d'obtenir avec les produits disponibles.

Plus spécifiquement, la présente invention concerne les composés de formule (I) : 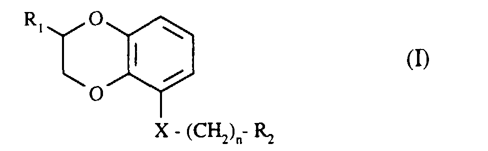 dans laquelle :
- X: représente un atome d'oxygène ou un groupement méthylène,
- n: représente 1, 2 ou 3,
- R₁: représente un atome d'hydrogène, un groupement aminocarbonyle ou un groupement hydroxyméthyle,
- R₂: représente un groupement : 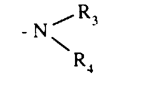 dans lequel :
soit R₃ représente un atome d'hydrogène ou un groupement alkyle (C₁-C₆) linéaire ou ramifié,
et R₄ représente :
* un groupement alkyle (C₁-C₄) linéaire ou ramifié
   - non substitué ou substitué par un groupement phényle (substitué ou non par un ou plusieurs atomes d'halogène ou groupements alkyle (C₁-C₄) linéaire ou ramifié, alkoxy (C₁-C₄) linéaire ou ramifié, trihalogénométhyle ou hydroxy), à la condition que dans ces cas R₁ soit différent d'un atome d'hydrogène,
   - ou substitué par un groupement benzoylamino (substitué ou non sur le noyau phényle par un ou plusieurs atomes d'halogène, ou groupements alkyle (C₁-C₄) linéaire ou ramifié, alkoxy (C₁-C₄) linéaire ou ramifié, trihalogénométhyle ou hydroxy),
* ou, un groupement benzoyl (substitué ou non par un ou plusieurs atomes d'halogène ou groupements alkyle (C₁-C₄) linéaire ou ramifié, alkoxy (C₁-C₄) linéaire ou ramifié, trihalogénométhyle ou hydroxy),
soit R₃ et R₄ forment avec l'atome d'azote sur lequel ils sont attachés un groupement : 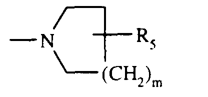 dans lequel:
m représente 1, 2 ou 3,
R₅ représente un groupement benzoyle (substitué ou non par un ou plusieurs atomes d'halogène ou groupements alkyle (C₁-C₄) linéaire ou ramifié, alkoxy (C₁-C₄) linéaire ou ramifié, hydroxy ou trihalogénométhyle),
benzoylméthyle (substitué ou non sur le noyau phényle par un ou plusieurs atomes d'halogène ou groupements alkyle (C₁-C₄) linéaire ou ramifié, alkoxy (C₁-C₄) linéaire ou ramifié, hydroxy ou trihalogénométhyle),
2-oxo-(3H)-benzimidazol-1-yle-,
1H-indazol-3-ylméthyle (substitué ou non sur le noyau phényle par un atome d'halogène),
ou 1,2-benzisoxazol-3-ylméthyle (substitué ou non sur le noyau phényle par un atome d'halogène),
leurs énantiomères, diastéréoisomères et épimères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend également au procédé de préparation des composés de formule (I).
Le procédé de préparation des composés de formule (I) tels que X représente un atome d'oxygène et R₁ représente un atome d'hydrogène est caractérisé en ce que l'on utilise comme produit de départ un composé de formule (II) : 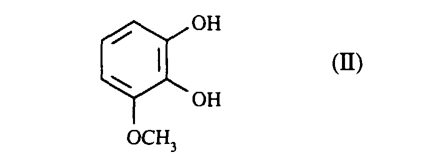 que l'on fait réagir avec du 1,2-dibromoéthane en présence de carbonate de potassium et de poudre de cuivre en milieu alcoolique,
pour conduire au composé de formule (III) : 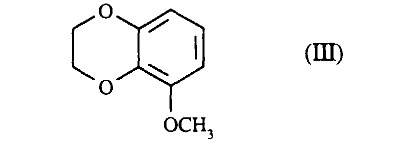 que l'on transforme, en milieu acide, en composé de formule (IV) : 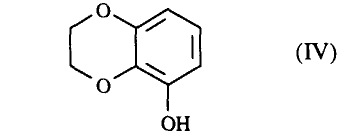 composé de formule (IV) que l'on condense :
*ou bien* sur un composé de formule (V), après réaction avec de l'éthanolate de sodium :

Br-(CH₂)ₙ-Cl (V)

dans laquelle n a la même signification que dans la formule (I),
pour conduire au composé de formule (VI) : 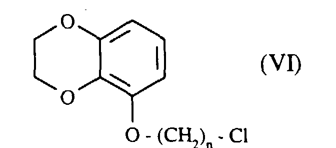 dans laquelle n a la même signification que dans la formule (I),
que l'on fait ensuite réagir sur une amine de formule (VII), dont on a éventuellement séparé les isomères : 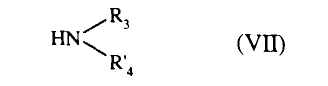 dans laquelle R'₄ a la même signification que R₄ à l'exception du cas où R₄ représente un groupement benzoyl substitué ou non,
pour conduire au composé de formule (I/a), cas particulier des composés de formule (I) : 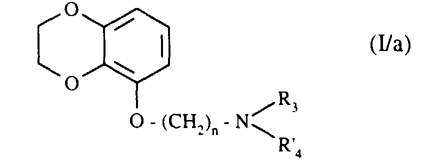 dans laquelle n, R₃ et R'₄ ont la même signification que précédemment,
*ou bien* sur un nitrile de formule (VIII), en milieu basique,

Br-(CH₂)ₙ₋₁-CN (VIII)

dans laquelle n a la même signification que dans la formule (I),
pour conduire au composé de formule (IX) : 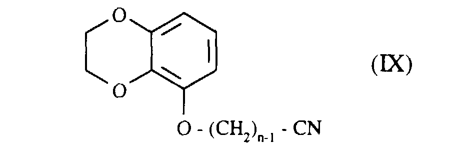 dans laquelle n a la même signification que dans la formule (I),
que l'on réduit à l'aide d'hydrure de lithium aluminium,
pour conduire au composé de formule (X) : 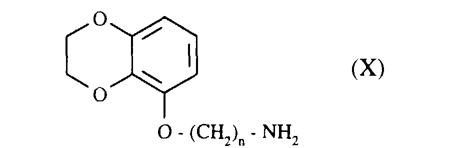 dans laquelle n a la même signification que dans la formule (I),
que l'on fait réagir sur un chlorure d'acide de formule (XI) :

Cl-CO-R''₄ (XI)

dans laquelle R''₄ représente un groupement phényl (substitué ou non par un ou plusieurs atomes d'halogène ou groupements alkyle (C₁-C₄) linéaire ou ramifié, alkoxy (C₁-C₄) linéaire ou ramifié, trihalogénométhyle ou hydroxy),
pour conduire au composé de formule (I/b), cas particulier des composés de formule (I) : 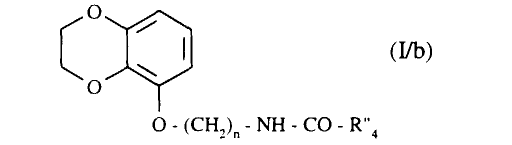 dans laquelle n et R''₄ ont la même signification que précédemment,
composé de formule (I/b) qui peut subir, si on le désire, une réduction en présence de borohydrure de tétrabutylammonium,
pour conduire au composé de formule (I/c), cas particulier des composés de formule (I) : 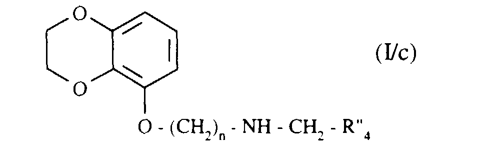 dans laquelle n et R''₄ ont la même signification que précédemment,
composé de formule (I/a), (I/b) ou (I/c) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

Le procédé de préparation des composés de formule (I) tels que X représente un atome d'oxygène et R₁ représente un groupement aminocarbonyl ou hydroxyméthyle est caractérisé en ce que l'on utilise comme produit de départ un composé de formule (XII) : 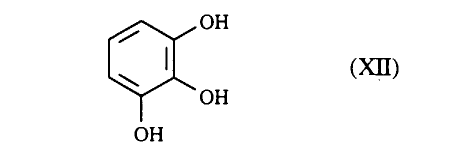 que l'on fait réagir avec le 2,3-dibromopropionate d'éthyle, en milieu basique,
pour conduire au composé de formule (XIII) : 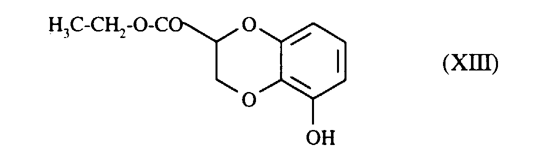 que l'on fait réagir, si on le souhaite, dans une solution ammoniacale,
pour conduire au composé de formule (XIV) : 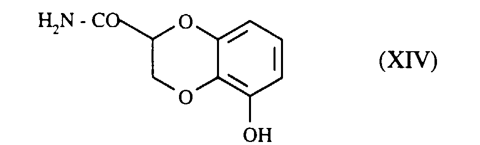 composé de formule (XIII) ou (XIV) que l'on condense :
*ou bien* sur un composé de formule (V) :

Br-(CH₂)ₙ-Cl (V)

dans laquelle n a la même signification que dans la formule (I),
pour conduire respectivement aux composés de formule (XV) ou (XVI) : 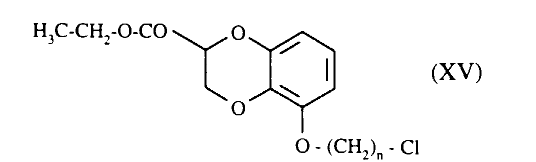 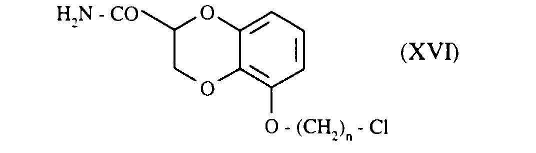 dans lesquelles n a la même signification que dans la formule (I),
que l'on fait réagir respectivement sur une amine de formule (VII), dont on a éventuellement séparé les isomères : 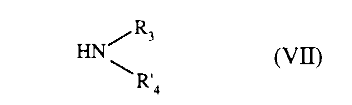 dans laquelle R'₄ a la même signification que R₄, à l'exception du cas où R₄ représente un groupement benzoyl substitué ou non,
pour conduire aux composés de formule (XVII) ou (I/d), cas particulier des composés de formule (I) : 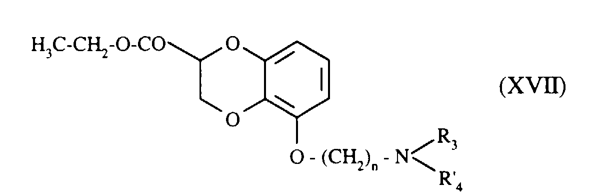 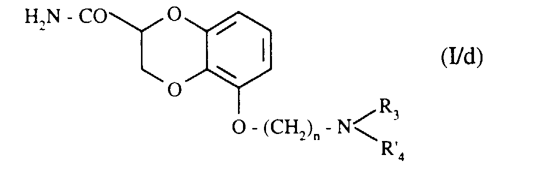 dans lesquelles n, R₃ et R'₄ ont la même signification que précédemment,
composé de formule (XVII) que l'on réduit en présence d'hydrure de lithium aluminium, pour conduire au composé de formule (I/e), cas particulier des composés de formule (I) : 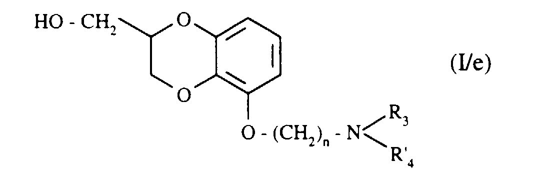 dans laquelle n, R₃ et R'₄ ont la même signification que dans la formule (I),
*ou bien*, sur un nitrile de formule (VIII), en milieu basique,

Br-(CH₂)ₙ₋₁- CN (VIII)

dans laquelle n a la même signification que dans la formule (I),
pour conduire respectivement aux composés de formule (XVIII) ou (XIX) : 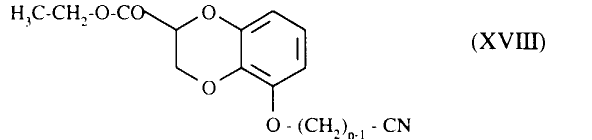 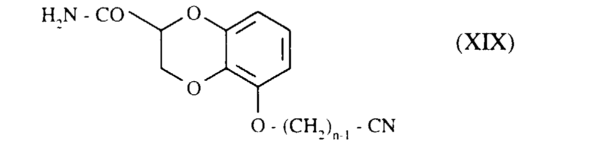 dans lesquelles n a la même signification que dans la formule (I),
que l'on réduit à l'aide d'hydrure de lithium aluminium,
pour conduire respectivement aux composés de formule (XX) ou (XXI) : 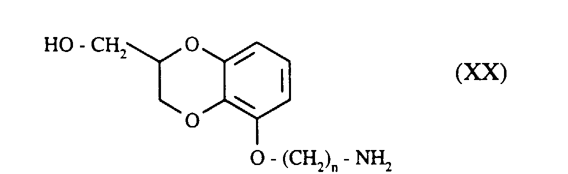 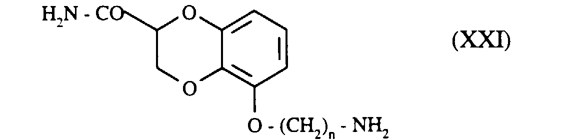 dans lesquelles n a la même signification que dans la formule (I),
que l'on fait réagir, respectivement, sur un chlorure d'acide de formule (XI) :

Cl-CO-R''₄ (XI)

dans laquelle R''₄ représente un groupement phényl (substitué ou non par un ou plusieurs atomes d'halogène ou groupements alkyle (C₁-C₄) linéaire ou ramifié, alkoxy (C₁-C₄) linéaire ou ramifié, trihalogénométhyle ou hydroxy),
pour conduire respectivement aux composés de formule (I/f) ou (I/g), cas particuliers des composés de formule (I) : 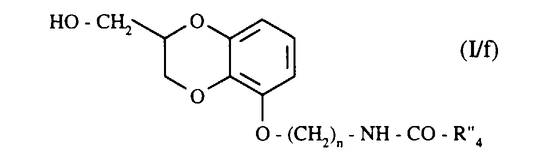 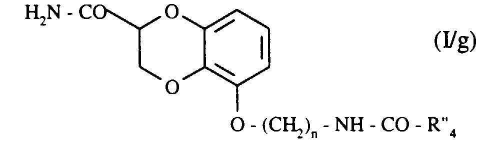 dans lesquelles n et R''₄ ont la même signification que précédemment,
composés de formule (I/f) ou (I/g), qui peuvent subir, si on le désire, une réduction en présence de borohydrure de tétrabutylammonium,
pour conduire respectivement aux composés de formule (I/h) ou (I/i), cas particuliers des composés de formule (I) : 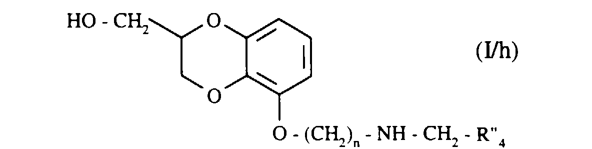 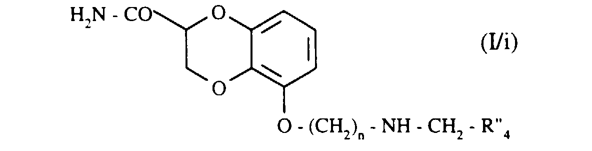 dans lesquelles n et R''₄ ont la même signification que précédemment,
composé de formule (I/d), (I/e), (I/f), (I/g), (I/h) ou (I/i) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

Le procédé de préparation des composés de formule (I) tels que X représente un groupement méthylène est caractérisé en ce que l'on utilise comme produit de départ un composé de formule (XXII) (obtenu à partir du 2,3-dihydroxybenzaldéhyde selon le procédé décrit dans J. Med.Chem., 26, 193, 1989): 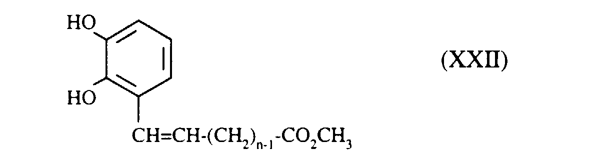 dans laquelle n a la même signification que dans la formule (I), qui subit une hydrogénation catalytique pour conduire au composé de formule (XXIII): 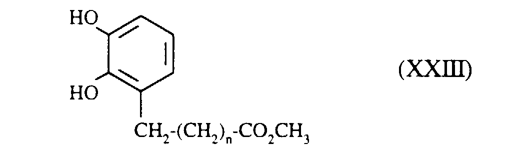 dans laquelle n a la même signification que dans la formule (I),
que l'on réduit en présence d'hydrure de lithium aluminium, pour conduire au composé de formule (XXIV) : 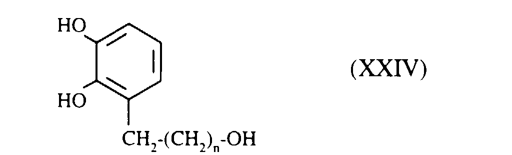 dans laquelle n a la même signification que dans la formule (I),
que l'on fait réagir, avec le 1,2-dibromoéthane ou avec le 2,3-dibromopropionate d'éthyle, selon le dérivé de formule (I), que l'on souhaite obtenir,
pour conduire au composé de formule (XXV) : 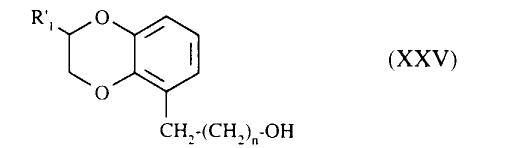 dans laquelle R'₁ représente un atome d'hydrogène ou un groupement éthoxycarbonyle, composé de formule (XXV) (qui lorsque R'₁ représente un groupement éthoxycarbonyle peut subir, si on le souhaite, l'action d'une solution ammoniacale, pour conduire à l'amide correspondant),
que l'on met ensuite en réaction avec du tétrabromure de carbone en présence de triphénylphosphine,
pour conduire au composé de formule (XXVI), 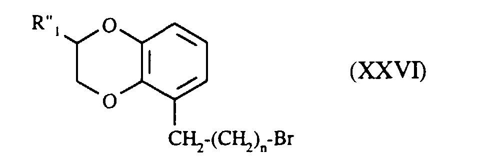 dans laquelle n a la même signification que dans la formule (I), et R''₁ représente un atome d'hydrogène, un groupement éthoxycarbonyle ou aminocarbonyle,
que l'on fait ensuite réagir avec une amine de formule (VII), dont on a éventuellement séparé
les isomères : 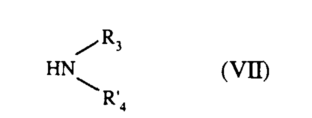 dans laquelle R'₄ a la même signification que R₄, à l'exception du cas où R₄ représente un groupement benzoyl substituté ou non,
pour conduire au composé de formule (I/j), cas particulier des composés de formule (I) (après transformation, en présence d'hydrure de lithium aluminium, du groupement R''₁ quand il représente un groupement éthoxycarbonyle, en groupement hydroxyméthyle) : 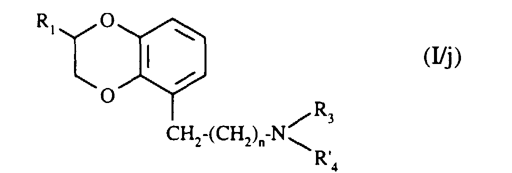 dans laquelle R₁, n, R₃ et R'₄ ont la même signification que précédemment,
composé de formule (I/j):
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 20 µg et 2 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

Les préparations A, B et C ne conduisent pas aux composés de l'invention mais à des intermédiaires de synthèse utiles dans la préparation des composés de formule (I).

### Préparation A : N-Propyl-N-[2-(4-fluorobenzoylamino)éthyl]amine

### Stade A : N-(2-Bromoéthyl)-4-fluorobenzamide

A une suspension contenant 0,23 mole de bromhydrate de β-bromoéthylamine dans 200 ml de chloroforme agitée à 5°C, est ajoutée, goutte à goutte, une solution contenant 0,21 mole de chlorure de l'acide 4-fluorobenzoïque dans 50 ml de chloroforme puis une solution contenant 0,46 mole de triéthylamine dans 100 ml de chloroforme. L'ensemble est alors agité 1 heure à température ambiante puis 4 heures à reflux. Après refroidissement et évaporation du solvant, le résidu est repris par 200 ml d'éther. Après filtration des sels et concentration du solvant, le produit attendu est obtenu par purification du résidu par chromatographie sur colonne de silice en utilisant comme éluant un mélange dichlorométhane/acétone (98/2).

### Stade B : N-Propyl-N-[2-(4-fluorobenzoylamino)éthyl]amine

Le produit obtenu au stade précédent en solution dans 40 ml de propylamine est porté à reflux pendant 4 heures. Après refroidissement et distillation de la propylamine en excès, le produit attendu est obtenu après purification par chromatographie sur colonne de silice en utilisant comme éluant un mélange dichlorométhane/méthanol/ammoniaque (90/10/1).

### Préparation B : 3-[(6-Fluoro-1,2-benzisoxazol-3-yl)méthyl]pyrrolidine

### Stade A : 1-Benzyl-3-[2-(2,4-difluorophényl)-2-hydroxyéthyl]pyrrolidine

A 65°C, une solution contenant 0,38 mole de 1-benzyl-3-chlorométhylpyrrolidine dans 200 ml de tétrahydrofurane (THF) est additionnée à une suspension contenant 9,3 g de magnésium dans 100 ml de THF. L'ensemble est porté à reflux et après disparition du métal, puis refroidissement, cette solution est additionnée à une solution contenant 0,38 mole du 2,4-difluorobenzaldéhyde dans 300 ml de THF. L'ensemble est maintenu 12 heures à température ambiante puis hydrolysé par 220 ml d'une solution saturée de chlorure d'ammonium. Après extraction au dichlorométhane puis évaporation, l'huile résiduelle est purifiée par chromatographie sur colonne de silice en utilisant comme éluant un mélange dichlorométhane/méthanol/ammoniaque (98/2/0,2) et conduit au produit attendu.

### Stade B : 1-Benzyl-3-(2,4-difluorobenzoylméthyl)pyrrolidine

A 0°C, sous agitation, 67 g de trioxyde de chrome sont ajoutés à 700 ml de pyridine puis 15 minutes après, 0,214 mole du composé décrit au stade précédent en solution dans 150 ml de pyridine. L'ensemble est maintenu 12 heures à température ambiante, évaporé et le résidu est repris par 500 ml d'eau et 500 ml de dichlorométhane. Le précipité qui se forme est filtré et lavé par du dichlorométhane. Les phases organiques sont réunies, lavées par de la soude 1N, par une solution saturée de chlorure de sodium puis séchées et évaporées. L'huile résiduelle est purifiée par chromatographie sur colonne de silice en utilisant comme éluant un mélange dichlorométhane/méthanol/ammoniaque (98/2/0,2) et conduit au produit attendu.

### Stade C : 1-Benzyl-3-[6-fluoro-1,2-benzisoxazol-3-yl)méthyl]pyrrolidine

0,19 mole du produit obtenu au stade précédent et 0,95 mole de chlorhydrate d'hydroxylamine dans 600 ml de pyridine sont portées à reflux 24 heures. Après concentration du solvant, le résidu est repris par du dichlorométhane. La phase organique est alors lavée à l'eau et évaporée. Le résidu est repris par 100 ml de diméthylformamide (DMF) et est additionné à une suspension contenant 4,3 g d'hydrure de sodium dans 50 ml de DMF. Après une heure d'agitation, le mélange réactionnel est hydrolysé puis extrait par du dichlorométhane. Après lavage à l'eau de la phase organique, évaporation du solvant, l'huile résiduelle est purifiée par chromatographie sur colonne de silice en utilisant comme éluant un mélange dichlorométhane/méthanol (97/3) et conduit au produit attendu.

### Stade D : 1-Ethoxycarbonyl-3-[6-fluoro-1,2-benzisoxazol-3-yl)méthyl]pyrrolidine

Un mélange contenant 80 mmoles du composé obtenu au stade précédent et 160 mmoles de chloroformiate d'éthyle dans 350 ml de toluène est porté à reflux 24 heures. Après refroidissement, lavage de la phase toluènique par de l'acide chlorhydrique 1N, puis par de la soude 1N et évaporation, on obtient le produit attendu.

### Stade E : 3-[(6-Fluoro-1,2-benzisoxazol-3-yl)méthyl]pyrrolidine

58 mmoles du composé obtenu au stade précédent et 250 ml d'acide bromhydrique à 48 % sont chauffés 1 heure à 90°C. Après concentration, le produit attendu est obtenu par relargage du résidu à l'aide de soude 5N.

### Préparation C : 3-[(6-Fluoro-1H-indazol-3-yl)méthyl]pyrrolidine

### Stade A : 1-Benzyl-[(6-fluoro-1H-indazol-3-yl)méthyl]pyrrolidine

64 mmoles du composé obtenu au stade B de la préparation B et 64 mmoles d'hydrate d'hydrazine dans 200 ml de 1-butanol sont portées 72 heures à reflux. Après refroidissement et évaporation, le résidu est repris par du dichlorométhane. La phase organique est lavée à l'eau et évaporée. L'huile résiduelle est purifiée par chromatographie sur colonne de silice en utilisant comme éluant un mélange dichlorométhane/méthanol/ammoniaque (96/4/0,4).

### Stade B : 3-[(6-Fluoro-1H-indazol-3-yl)méthyl]pyrrolidine

Le produit obtenu au stade précédent subit une débenzylation catalytique dans de l'éthanol, à 45°C, en utilisant comme catalyseur du palladium sur charbon à 10 %.

### EXEMPLE 1: 5-{2-[4-(4-Fluorobenzoyl)pipéridino]éthoxy}-1,4-benzodioxane, chlorhydrate

### Stade A : 5-Méthoxy-1,4-benzodioxane

Un mélange hétérogène contenant 1,42 moles de 3-méthoxycatecol, 1,57 moles de 1,2-dibromoéthane, 1,42 moles de carbonate de potassium et 4 g de poudre de cuivre dans 150 ml de glycérol est chauffé pendant 15 heures à 110°C. Après refroidissement, le mélange est versé dans 1 l d'eau. La phase aqueuse est extraite par de l'éther éthylique. Les phases éthérées sont alors lavées par de la soude 1N, séchées, évaporées et conduisent au produit attendu.

### Stade B : 5-Hydroxy-1,4-benzodioxane

Le composé obtenu au stade précédent est porté à reflux dans 500 ml d'acide bromhydrique à 48 % et 800 ml d'acide acétique glacial. Après refroidissement et concentration, le résidu est repris par 1 l d'eau et la phase aqueuse extraite par de l'éther éthylique. Les phases éthérées sont réunies et évaporées. Le produit attendu est obtenu après purification par chromatographie sur colonne de silice en utilisant comme éluant du dichlorométhane.

### Stade C : 5-(2-Chloroéthoxy)-1,4-benzodioxane

A 500 ml d'une solution 1N d'éthanolate de sodium, est ajoutée, goutte à goutte, une solution contenant 500 mmoles du composé obtenu au stade précédent dans 150 ml d'éthanol. L'ensemble est agité 1 heure et une solution contenant 500 mmoles de 1-bromo-2-chloroéthane dans 150 ml d'éthanol est ajoutée au mélange précédent. L'ensemble est alors porté 24 heures à reflux. Après refroidissement, filtration des sels et évaporation, le résidu est repris dans de l'éther isopropylique et le produit attendu qui précipite est filtré.
*Point de fusion : 73°C*

### Stade D : 5-{2-[4-(4-Fluorobenzoyl)pipéridino]éthoxy}-1,4-benzodioxane, chlorhydrate

Un mélange contenant 21 mmoles du produit obtenu au stade précédent, 17 mmoles de 4-(4-fluorobenzoyl)pipéridine, 21 mmoles de carbonate de potassium et quelques mg d'iodure de potassium dans 50 ml de 4-méthyl-2-pentanone est porté à reflux pendant 18 heures. Après refroidissement, le milieu réactionnel est additionné de 50 ml d'eau et la phase aqueuse est extraite par du dichlorométhane. Les phases organiques sont réunies, lavées, séchées et évaporées. Le produit attendu, sous forme de base, est obtenu après purification du résidu par chromatographie sur colonne de silice en utilisant comme éluant un mélange dichlorométhane/éthanol (98/2). Il est transformé en chlorhydrate correspondant, par dilution dans de l'éther éthylique et addition goutte à goutte de la quantité stoechiométrique d'éthanol chlorhydrique 10N. Le chlorhydrate est alors filtré, lavé à l'éther et séché.
*Point de fusion : 152°C*

| *Microanalyse élémentaire :* | | | | |
|---|---|---|---|---|
| | *C %* | *H %* | *N %* | *Cl %* |
| *calculé* | *62,63* | *5,97* | *3,32* | *8,40* |
| *trouvé* | *62,49* | *5,95* | *3,18* | *8,47* |

Les composés des exemples 2 à 8 ont été préparés selon le procédé décrit dans l'exemple 1 en utilisant les produits de départ correspondants.

### EXEMPLE 2 : 5-{2-[3-(4-Fluorobenzoylméthyl)pyrrolidin-1-yl]éthoxy}-1,4-benzodioxane, chlorhydrate

Stades A, B, C : identiques aux stades A, B, C de l'exemple 1.

### Stade D : Remplacer la 4-(4-fluorobenzoyl)pipéridine par la 3-(4-fluorobenzoylméthyl) pyrrolidine.

*Point de fusion : 134°C*

| *Microanalyse élémentaire :* | | | | |
|---|---|---|---|---|
| | *C %* | *H %* | *N %* | *Cl %* |
| *calculé* | *62,63* | *5,97* | *3,32* | *8,40* |
| *trouvé* | *62,64* | *6,06* | *3,16* | *8,52* |

### EXEMPLE 3 : 5-{2-[4-(2-Oxo-(3H)-benzimidazol-1-yl)pipéridino]éthoxy}-1,4-benzodioxane, chlorhydrate

Stades A, B, C : identiques aux stades A, B et C de l'exemple 1.

### Stade D : Remplacer la 4-(4-fluorobenzoyl)pipéridine par le1-(pipéridin-4-yl)-2-(3H)-benzimidazolone

*Point de fusion : 155°C*

| *Microanalyse élémentaire :* | | | | |
|---|---|---|---|---|
| | *C %* | *H %* | *N %* | *Cl %* |
| *calculé* | *61,18* | *6,07* | *9,73* | *8,21* |
| *trouvé* | *61,07* | *6,1* | *9,71* | *8,14* |

### EXEMPLE 4 : 5-{2-[N-propyl-N-[2-(4-Fluorobenzoylamino)éthyl]amino]éthoxy}-1,4-benzodioxane, chlorhydrate

Stades A, B, C : identiques aux stades A, B, C de l'exemple 1.

### Stade D : Remplacer la 4-(4-fluorobenzoyl)pipéridine par l'amine décrite dans la préparation A.

*Point de fusion : 147°C*

| *Microanalyse élémentaire :* | | | | |
|---|---|---|---|---|
| | *C %* | *H %* | *N %* | *Cl %* |
| *calculé* | *60,20* | *6,43* | *6,38* | *8,08* |
| *trouvé* | *59,71* | *6,53* | *6,33* | *8,27* |

### EXEMPLE 5 : 5-{2-[3-(4-Fluorobenzoylméthyl)pyrrolidin-1-yl]éthoxy}-1,4-benzodioxane, chlorhydrate, isomère (+)

Stades A, B, C : identiques aux stades A, B, C de l'exemple 1.

### Stade D : Remplacer la 4-(4-fluorobenzoyl)pipéridine par l'isomère (+) de la 3-(4-fluorobenzoylméthyl)pyrrolidine obtenu selon le procédé décrit dans le brevet EP 389352.

Le chlorhydrate est obtenu par salification de la base dans un mélange acétone/éther éthylique (50/50) avec un équivalent d'acide chlorhydrique dans l'éthanol.
*Point de fusion : 120-122°C*
*Pouvoir rotatoire : [*α*]*_{*D*}^{*21*} = + *4,15*° *(c = 1 %, méthanol)*

| *Microanalyse élémentaire :* | | | | |
|---|---|---|---|---|
| | *C %* | *H %* | *N %* | *Cl %* |
| *calculé* | *62,63* | *5,97* | *3,32* | *8,40* |
| *trouvé* | *62,65* | *5,89* | *3,31* | *8,41* |

### EXEMPLE 6 : 5-{2-[3-(4-Fluorobenzoylméthyl)pyrrolidin-1-yl]éthoxy}-1,4-benzodioxane, chlorhydrate, isomère (-)

Stades A, B, C : identiques aux stades A, B, C de l'exemple 1.

### Stade D : Remplacer la 4-(4-fluorobenzoyl)pipéridine par l'isomère (-) de la 3-(4-fluorobenzoylméthyl)pyrrolidine obtenu selon le procédé décrit dans le brevet EP 389352.

Le chlorhydrate est obtenu comme décrit dans l'exemple 5.
*Point de fusion : 120-122°C*
*Pouvoir rotatoire : [α]*_{*D*}^{*21*} : - *4,75° (c = 1 %, méthanol)*

| *Microanalyse élémentaire :* | | | | |
|---|---|---|---|---|
| | *C %* | *H %* | *N %* | *Cl %* |
| *calculé* | *62,63* | *5,97* | *3,32* | *8,40* |
| *trouvé* | *62,59* | *5,86* | *3,37* | *8,28* |

### EXEMPLE 7 : 5-{2-[3-[(6-Fluoro-1H-indazol-3-yl)méthyl]pyrrolidin-1-yl]éthoxy}-1,4-benzodioxane, chlorhydrate

Stades A, B, C : identiques aux stades A, B, C de l'exemple 1.

### Stade D : Remplacer la 4-(4-fluorobenzoyl)pipéridine par la 3-[(6-fluoro-1H-indazol-3-yl)méthyl]pyrrolidine décrite dans la préparation C.

La purification du produit attendu sous forme de base est réalisée par chromatographie sur colonne de silice en utilisant comme éluant un mélange dichlorométhane/méthanol/ ammoniaque (95/5/0,5).

| *Microanalyse élémentaire :* | | | | |
|---|---|---|---|---|
| | *C %* | *H %* | *N %* | *Cl %* |
| *calculé* | *61,26* | *5,78* | *9,75* | *7,40* |
| *trouvé* | *61,33* | *6,05* | *9,14* | *7,35* |

### EXEMPLE 8 : 5-{2-[3-[(6-Fluoro-1,2-benzisoxazol-3-yl)méthyl]pyrrolidin-1-yl]éthoxy}-1,4-benzodioxane, chlorhydrate

Stades A, B, C : identiques aux stades A, B, C de l'exemple 1.

### Stade D : Remplacer la 4-(4-fluorobenzoyl)pipéridine par la 3-[(6-fluoro-1,2-benzisoxazol-3-yl)méthyl]pyrrolidine décrite dans la préparation B.

La purification du produit obtenu sous forme de base est réalisée par chromatographie sur colonne de silice en utilisant comme éluant un mélange dichlorométhane/méthanol (97/3).

| *Microanalyse élémentaire :* | | | |
|---|---|---|---|
| | *C %* | *H %* | *N %* |
| *calculé* | *60,76* | *5,56* | *6,44* |
| *trouvé* | *61,21* | *5,54* | *6,49* |

### EXEMPLE 9 : 5-{2-[4-(4-Fluorobenzoyl)pipéridino]éthoxy}-1,4-benzodioxane-2-carboxamide, chlorhydrate

### Stade A : Ester éthylique de l'acide 5-hydroxy-1,4-benzodioxane-2-carboxylique

A une solution contenant 0,8 mole de pyrogallol dans 1 l d'acétone sont ajoutées 2,4 moles de carbonate de potassium puis goutte à goutte 2,4 moles de 2,3-dibromopropionate d'éthyle. L'ensemble est alors porté 15 heures à reflux. Après refroidissement, filtration du précipité, lavage de celui-ci à l'acétone, le filtrat est récupéré et concentré. Le résidu est repris à l'éther et après filtration de la phase éthérée, celle-ci est évaporée et conduit au produit attendu.

### Stade B : 5-Hydroxy-1,4-benzodioxane-2-carboxamide

Un mélange contenant 350 mmoles du produit obtenu au stade précédent dans 1,3 l d'une solution ammoniacale à 28 % est agité 18 heures à température ambiante. Après concentration du milieu réactionnel, le résidu est repris par 300 ml d'éther. Le produit attendu est obtenu après filtration du précipité formé et séché.

### Stade C : 5-(2-Chloroéthoxy)-1,4-benzodioxane-2-carboxamide

Le produit attendu est obtenu selon le procédé décrit au stade C de l'exemple 1 à partir du composé obtenu au stade précédent.

### Stade D : 5-{2-[4-(4-Fluorobenzoyl)pipéridino]éthoxy}-1,4-benzodioxane-2-carboxamide, chlorhydrate

Le produit attendu est obtenu selon le procédé décrit au stade D de l'exemple 1 à partir du composé décrit au stade précédent.
*Point de fusion : 215°C*

| *Microanalyse élémentaire :* | | | | |
|---|---|---|---|---|
| | *C %* | *H %* | *N %* | *Cl %* |
| *calculé* | *59,42* | *5,64* | *6,03* | *7,63* |
| *trouvé* | *59,39* | *5,80* | *5,83* | *7,59* |

### EXEMPLE 10: 2-Hydroxyméthyl-5-[2-(N,N-dipropylamino)éthoxy]-1,4-benzodioxane, oxalate

### Stade A : Ester éthylique de l'acide 5-(2-chloroéthyl)-1,4-benzodioxane-2-carboxylique

Le produit attendu est obtenu à partir du composé décrit au stade A de l'exemple 9 selon le procédé décrit au stade C de l'exemple 1.

### Stade B : Ester éthylique de l'acide 5-[2-(N,N-dipropylamino)éthoxy]-1,4-benzodioxane-2-carboxylique

50 mmoles du composé obtenu au stade précédent dans 50 ml de dipropylamine sont portés à 150°C pendant 10 heures. Après refroidissement, le milieu est concentré sous vide et le produit attendu est obtenu après purification du résidu par chromatographie sur colonne de silice en utilisant comme éluant un mélange dichlorométhane/éthanol/ammoniaque (90/10/1).

### Stade C : 2-Hydroxyméthyl-5-[2-(N,N-dipropylamino)éthoxy]-1,4-benzodioxane, oxalate

A une suspension contenant 13,7 mmoles d'hydrure de lithium aluminium dans 100 ml d'éther, agitée à température ambiante sous atmosphère d'azote, est ajoutée, goutte à goutte, une solution contenant 12 mmoles du produit obtenu au stade précédent dans 50 ml d'éther. Le mélange est maintenu 1 heure sous agitation et est hydrolysé par une solution saturée de chlorure d'ammonium. La phase organique est récupérée, lavée à l'eau, séchée et évaporée et conduit au produit attendu sous forme de base. A celui-ci, dilué dans 50 ml d'éthanol, est ajouté 1 équivalent d'acide oxalique puis après 5 minutes d'agitation 50 ml d'éther isopropylique. L'oxalate est obtenu par filtration et séché.
*Point de fusion : 148°C*

### EXEMPLE 11: 5-[2-(2,3,4-Triméthoxybenzoylamino)éthoxy]-1,4-benzodioxane

### Stade A : 5-Cyanométhoxy-1,4-benzodioxane

A une suspension, sous agitation, contenant 0,38 mole du composé obtenu au stade B de l'exemple 1 et 1,14 moles de carbonate de potassium dans 500 ml d'acétone, est ajoutée, goutte à goutte, une solution contenant 0,76 mole de bromoacétonitrile dans 100 ml d'acétone. Le milieu est alors maintenu sous agitation 18 heures. Après filtration des sels et évaporation, on obtient le produit attendu.

### Stade B : 5-(2-Aminoéthoxy)-1,4-benzodioxane

Le produit attendu est obtenu selon le procédé décrit au stade C de l'exemple 10 à partir du produit décrit au stade précédent.

### Stade C : 5-[2-(2,3,4-Triméthoxybenzoylamino)éthoxy]-1,4-benzodioxane

A une solution, sous agitation, à température ambiante, contenant 4,7 mmoles du produit obtenu au stade précédent dans 20 ml de dichlorométhane est ajoutée goutte à goutte une solution contenant 10,5 mmoles du chlorure de l'acide 3,4,5-triméthoxybenzoïque dans 20 ml de dichlorométhane puis 10,5 mmoles de triéthylamine dans 20 ml de dichlorométhane. Le mélange est alors agité 2 heures à température ambiante. Après filtration des sels, lavage du milieu par de l'eau, la phase organique est séchée et évaporée. Le résidu repris à l'éther conduit au produit attendu qui précipite et qui est recristallisé dans de l'éthanol.
*Point de fusion : 128°C*

| *Microanalyse élémentaire :* | | | |
|---|---|---|---|
| | *C %* | *H %* | *N %* |
| *calculé* | *61,69* | *5,95* | *3,60* |
| *trouvé* | *61,26* | *5,89* | *3,70* |

### EXEMPLE 12: 2-Hydroxyméthyl-5-[2-(4-Fluorobenzoylamino)éthoxy]-1,4-benzodioxane

### Stade A : Ester éthylique de l'acide 5-cyanométhyl-1,4-benzodioxane-2-carboxylique

Le produit attendu a été obtenu selon le procédé décrit au stade A de l'exemple 11 à partir du composé décrit au stade A de l'exemple 9 et du bromoacétonitrile.

### Stade B : 2-Hydroxyméthyl-5-(2-aminoéthoxy)-1,4-benzodioxane

Le produit attendu a été obtenu selon le procédé décrit au stade C de l'exemple 10 à partir du composé décrit au stade précédent en utilisant 2 équivalents d'hydrure de lithium aluminium.

### Stade C : 2-Hydroxyméthyl-5-[2-(4-Fluorobenzoylamino)éthoxy]-1,4-benzodioxane

Le produit attendu a été obtenu selon le procédé décrit au stade C de l'exemple 11 à partir du composé décrit au stade précédent et du chlorure de l'acide 4-fluorobenzoïque.
*Point de fusion : 124°C*

| *Microanalyse élémentaire :* | | | |
|---|---|---|---|
| | *C %* | *H %* | *N %* |
| *calculé* | *62,24* | *5,22* | *4,03* |
| *trouvé* | *62,13* | *5,30* | *3,88* |

### Exemple 13: 5-[2-(3,4,5-Triméthoxybenzylamino)éthoxy]-1,4-benzodioxane, chlorhydrate

6,3 mmoles du composé obtenu au stade C de l'exemple 11 et 19 mmoles de borohydrure de tétrabutylammonium sont portées à reflux dans 100 ml de dichlorométhane pendant 24 heures. Après évaporation du solvant, le milieu réactionnel est hydrolysé par 50 ml d'acide chlorhydrique à 10 % à température ambiante. Le mélange est alors porté à reflux une heure. Après extraction au dichlorométhane, la phase organique est lavée à la soude, séchée et concentrée sous vide. Le produit attendu, sous forme de base, est alors obtenu après purification du résidu par chromatographie sur colonne de silice en utilisant comme éluant un mélange dichlorométhane/méthanol (97/3). Après dilution dans de l'éther et addition stoechiométrique d'éther chlorhydrique 6N, le chlorhydrate correspondant est obtenu.
*Point de fusion : 149°C*

| *Microanalyse élémentaire :* | | | | |
|---|---|---|---|---|
| | *C %* | *H %* | *N %* | *Cl %* |
| *calculé* | *58,32* | *6,36* | *3,40* | *8,61* |
| *trouvé* | *58,02* | *6,35* | *3,45* | *8,44* |

### EXEMPLE 14 : 5-{3-[3-(4-Fluorobenzoylméthyl)pyrrolidin-1-yl]propyl}-1,4-benzodioxane, chlorhydrate

### Stade A : Ester méthylique de l'acide 3-(2,3-dihydroxyphényl)propionique

Le produit attendu est obtenu par hydrogénation catalytique, à pression et température ambiantes, dans du méthanol, en présence de palladium sur charbon à 10 % comme catalyseur, de l'ester méthylique de l'acide (2,3-dihydroxyphényl)cinnamique (obtenu à partir de 2,3-dihydroxybenzaldéhyde selon le mode opératoire décrit dans J. Het. Chem., 26, 193, 1989).

### Stade B : 3-(2,3-Dihydroxyphényl)propanol

Le produit attendu est obtenu selon le procédé décrit au stade C de l'exemple 10, à partir du composé obtenu au stade précédent.

### Stade C : 5-(3-Hydroxypropyl)-1,4-benzodioxane

Le produit attendu est obtenu selon le procédé décrit au stade A de l'exemple 1 à partir du composé obtenu au stade précédent.

### Stade D : 5-(3-Bromopropyl)-1,4-benzodioxane

A une solution agitée à 8°C contenant 36 mmoles du composé obtenu au stade précédent et 58,4 mmoles de tétrabromure de carbone dans 150 ml de diméthylformamide, sont ajoutées, par petites fractions, 58,4 mmoles de triphénylphosphine. Le mélange est agité 2 heures à 8°C puis versé sur 300 ml d'eau glacée. Le milieu est extrait à l'éther et la phase éthérée, séchée et évaporée. Le produit attendu est alors obtenu par purification du résidu par chromatographie sur colonne de silice en utilisant comme éluant le dichlorométhane.

### Stade E : 5-{3-[3-(4-Fluorobenzoylméthyl)pyrrolidin-1-yl]propyl}-1,4-benzodioxane, chlorhydrate

Le produit attendu est obtenu selon le procédé décrit au stade D de l'exemple 1 à partir du composé obtenu au stade précédent et de 3-(4-fluorobenzoylméthyl)pyrrolidine.

| *Microanalyse élémentaire :* | | | | |
|---|---|---|---|---|
| | *C %* | *H %* | *N %* | *Cl %* |
| *calculé* | *65,79* | *6,48* | *3,34* | *8,44* |
| *trouvé* | *65,11* | *6,11* | *4,03* | *8,32* |

### EXEMPLE 15: 5-{2-[4-(4-Fluorobenzoyl)perhydroazépin-1-yl]éthoxy}-1,4 benzodioxane, chlorhydrate

Porter pendant 24 heures à 100°C un mélange de 5 g de 5-(2-chloroéthoxy)- 1,4 benzodioxane, 7,8 g de bromhydrate de 4-(4-fluorobenzoyl) perhydroazépine (décrit dans le brevet EP 389352), 1 g d'iodure de potassium et 6,5 g de carbonate de potassium dans 100 ml de diéthylcétone. Filtrer ensuite les sels minéraux et concentrer le filtrat sous vide. Le résidu est purifié par chromatographie sur colonne de silice avec un mélange éluant dichlorométhane/méthanol/ ammoniaque (97/3/0,3). La base obtenue est salifiée par de l'éthanol chlorhydrique dans un mélange acétone/éther.
*Point de fusion : 106-107°C*

| *Microanalyse élémentaire :* | | | | |
|---|---|---|---|---|
| | *C %* | *H %* | *N %* | *Cl %* |
| *calculé* | *63,37* | *6,24* | *3,21* | *8,13* |
| *trouvé* | *63,01* | *6,13* | *2,98* | *7,94* |

### Etude pharmacologique des composés de l'invention

### EXEMPLE 16: Profil réceptoriel des composés de l'invention

L'interaction de ces composés avec différents récepteurs a été déterminée en utilisant des études de fixation classiques comme celles décrites par M.J. MILLAN et coll. (J. Pharmacol. Exp. Ther., 268, 337-352, 1994 et Drug News Perspectives, 5, 397-406, 1992). Le profil réceptoriel des composés de l'invention est présenté dans le tableau ci-dessous. Les affinités des composés sont exprimées en pKi.

| Profil réceptoriel (pKi) | | | | | | |
|---|---|---|---|---|---|---|
| **Exemples** | **D**_{**1**} | **D**_{**2**} | **5-HT**_{**1A**} | **5-HT**_{**2A**} | **5-HT**_{**2C**} | **α**_{**1**} |
| ex. 2 | 6.60 | 7.36 | 8.31 | 8.40 | 8.10 | 8.61 |
| ex. 5 | 6.56 | 7.33 | 8.32 | 8.48 | 8.03 | 8.19 |
| ex. 6 | 6.69 | 7.27 | 8.44 | 8.62 | 8.18 | 8.93 |
| ex. 7 | NT | 7.27 | 8.77 | 7.66 | 7.03 | NT |
| ex. 8 | NT | 6.59 | 8.41 | 7.88 | 7.02 | NT |
| Halopéridol | 7.39 | 8.73 | 5.55 | 7.08 | 5.24 | 8.01 |
| Clozapine | 6.74 | 6.65 | 6.53 | 7.63 | 8.06 | 8.22 |
| NT = non testé | | | | | | |

### EXEMPLE 17: Propriétés antipsychotiques

Afin de déterminer les propriétés antipsychotiques des composés de l'invention, deux tests bien établis ont été utilisés dans lesquels l'ensemble des antipsychotiques sont cliniquement actifs comme l'ont montré A.Y. DEUTCH et coll. (Schizophrenia Research, 4, 121-156, 1991) et A.A. MENGENS et coll. (J. Pharmacol. Exp. Ther., 260, 160-167, 1992):
- inhibition de la verticalisation induite par un agoniste dopaminergique : l'apomorphine (selon le protocole décrit par P. PROTAIS et coll., Psychopharmacol., 50, 1-6, 1976),
- inhibition de l'activité motrice provoquée par l'amphétamine (qui augmente la libération des catécholamines) selon le protocole décrit par A.A. MENGENS cité plus haut.

Une activité dans ces tests semble refléter un blocage des voies dopaminergiques mésolimbiques dont une hyperactivité est supposée chez les schizophrènes comme décrit par P.C. WALDMEIER et coll. (Eur. J. Pharmacol., 55, 363-373, 1979) et A.Y. DEUTCH cité plus haut. Les activités des composés de l'invention ont été comparées à celles de l'halopéridol et de la clozapine.

### Test de verticalisation à l'apomorphine chez la souris

L'expérience a été effectuée sur des souris CF (Charles River) mâles de poids moyen 25 g. Trente minutes avant le début du test, chaque souris a été placée dans une cage cylindrique (⌀12 cm x 14 cm), à barreaux verticaux métalliques et couvercle plastique lisse, après avoir reçu une injection sous-cutanée de solvant ou de produit. A T₀, une solution d'apomorphine (0,75 mg/kg) ou de sérum physiologique a été administrée par voie sous-cutanée à l'animal, placé à nouveau dans la cage à barreaux. A T₁₀ (10 minutes) et à T₂₀ (20 minutes), un score a été attribué à chaque animal après une minute d'observation environ:
- score 0 (4 pattes au sol) ;
- score 1 (animal redressé, pattes avant sur les barreaux verticaux) ;
- score 2 (animal accroché par les 4 pattes sur les barreaux).

Le score total des 2 mesures représente alors la valeur de verticalisation de l'animal, utilisée pour l'analyse statistique.

### Test de l'activité motrice provoquée par l'amphétamine

Les rats Wistar, mâles (200-220 g) ont été placés en cages individuelles en polycarbonate transparent, la veille du test. L'activité locomotrice a été enregistrée à l'aide de cellules photoélectriques (Système Lablinc, Coulbourn) connectées par interface, à un microordinateur. Il y a deux faisceaux transversaux de cellules photoélectriques par cage. Le test a lieu sous lumière artificielle, à partir de 13 h, en période diurne. Le produit ou solvant a été administré à l'animal, par voie sous-cutanée, 30 minutes avant le début du test. A T₀, l'animal a reçu une injection intrapéritonéale de soluté physiologique ou de d-amphétamine (2,5 mg/kg) : l'activité motrice de l'animal (petits et grands déplacements) a été enregistrée de façon continue pendant 1 heure, de T₀ à T₆₀.

Les résultats de ces deux tests sont présentés dans le tableau ci-dessous :

| **Exemples** | **Verticalisation (Apomorphine)** | | **Locomotion (Amphétamine)** | |
|---|---|---|---|---|
| | **DI**_{**50**} **(mg/kg)** | **(95 % L.C.) (mg/kg)** | **DI**_{**50**} **(mg/kg)** | **(95 % L.C.) (mg/kg)** |
| Ex. 2 | 0.28 | (0.17-0.47) | 2.17 | (1.44-3.28) |
| Ex. 5 | 0.26 | (0.12-0.57) | 2.80 | (1.51-5.18) |
| Ex. 6 | 0.25 | (0.15-0.41) | 1.94 | (1.05-3.60) |
| Ex. 7 | 0.41 | (0.04-3.99) | NT | |
| Ex. 8 (*) | ≈ 0.63 | | NT | |
| Halopéridol | 0.013 | (0.008-0.022) | 0.043 | (0.03-0.06) |
| Clozapine | 2.22 | (1.70-2.90) | 12.85 | (9.25-17.86) |

| | | | | |
|---|---|---|---|---|
| DI₅₀ (95 % L.C.) = Dose Inhibitrice₅₀ (95 % Limites de Confiance) NT = non testé (*) La valeur précise n'a pu être déterminée | | | | |

### EXEMPLE 18: Catalepsie chez le rat

Afin de déterminer le potentiel des composés de l'invention à engendrer un syndrome de type extrapyramidal, la capacité à induire une catalepsie chez le rat a été examinée. Ce phénomène est dû à un antagonisme de la transmission dopaminergique nigrostriatale. Les activités des composés ont été comparées à celles de l'halopéridol et la clozapine.

### Protocole :

Les rats Wistar, mâles (220-240 g) ont été placés en cages individuelles et mis à jeun la veille du test.
Le test utilisé pour évaluer les propriétés cataleptogènes d'un produit consiste à placer chaque patte arrière de l'animal sur la patte avant située du même côté et à mesurer le temps en secondes pendant lequel l'animal a gardé cette position de "pattes croisées", jusqu'à 30 secondes comme décrit par P.C. WALDMEIER et coll. (Eur. J. Pharmacol., 55, 363-373, 1979).
Chaque animal a subi trois essais, à une minute d'intervalle ; la valeur moyenne des trois essais représente alors le temps de catalepsie de l'animal utilisé pour l'analyse statistique. Le produit à tester a été administré à l'animal, par voie sous-cutanée, 30 minutes avant le test.

Les résultats de l'induction de la catalepsie chez le rat sont présentés dans le tableau ci-dessous :

La dose cataleptogène efficace DE₅₀ est celle qui induit une catalepsie d'une durée moyenne de 15 secondes, c'est-à-dire 50 % de la durée maximale du test (30 secondes).

| **Exemples** | **Induction de la catalepsie** | |
|---|---|---|
| | **DE**_{**50**} **(mg/kg)** | **(95 % C.L.) (mg/kg)** |
| Ex. 2 | > 40.0 | |
| Ex. 5 | > 40.0 | |
| Ex. 6 | > 40.0 | |
| Halopéridol | 0.156 | (0.05-0.38) |
| Clozapine | > 40.0 | |
| DE₅₀ (95 % C.L.) = Dose Effective₅₀ (95 % Limites de Confiance) | | |

### EXEMPLE 19 : Composition pharmaceutique

Formule de préparation pour 1000 comprimés dosés à 0,1 mg

| | |
|---|---|
| Composé de l'exemple 2 | 100 mg |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 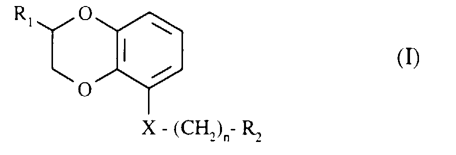 dans laquelle :
X représente un atome d'oxygène ou un groupement méthylène,
n représente 1, 2 ou 3,
R₁ représente un atome d'hydrogène, un groupement aminocarbonyle ou un groupement hydroxyméthyle,
R₂ représente un groupement : 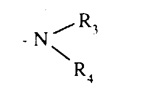 dans lequel :
soit R₃ représente un atome d'hydrogène ou un groupement alkyle (C₁-C₆) linéaire ou ramifié,
et R₄ représente :
* un groupement alkyle (C₁-C₄) linéaire ou ramifié
- non substitué ou substitué par un groupement phényle (substitué ou non par un ou plusieurs atomes d'halogène ou groupements alkyle (C₁-C₄) linéaire ou ramifié, alkoxy (C₁-C₄) linéaire ou ramifié, trihalogénométhyle ou hydroxy), à la condition que dans ces cas R₁ soit différent d'un atome d'hydrogène,
- ou substitué par un groupement benzoylamino (substitué ou non sur le noyau phényle par un ou plusieurs atomes d'halogène, ou groupements alkyle (C₁-C₄) linéaire ou ramifié, alkoxy (C₁-C₄) linéaire ou ramifié, trihalogénométhyle ou hydroxy),
* ou, un groupement benzoyl (substitué ou non par un ou plusieurs atomes d'halogène ou groupements alkyle (C₁-C₄) linéaire ou ramifié, alkoxy (C₁-C₄) linéaire ou ramifié, trihalogénométhyle ou hydroxy),
soit R₃ et R4 forment avec l'atome d'azote sur lequel ils sont attachés un groupement : 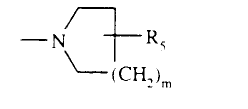 dans lequel :
m représente 1, 2 ou 3,
R₅ représente un groupement benzoyle (substitué ou non par un ou plusieurs atomes d'halogène ou groupements alkyle (C₁-C₄) linéaire ou ramifié, alkoxy (C₁-C₄) linéaire ou ramifié, hydroxy ou trihalogénométhyle),
benzoylméthyle (substitué ou non sur le noyau phényle par un ou plusieurs atomes d'halogène ou groupements alkyle (C₁-C₄) linéaire ou ramifié, alkoxy (C₁-C₄) linéaire ou ramifié, hydroxy ou trihalogénométhyle),
2-oxo-(3H)-benzimidazol-1-yle-,
1H-indazol-3-ylméthyle (substitué ou non sur le noyau phényle par un atome d'halogène),
ou 1,2-benzisoxazol-3-ylméthyle (substitué ou non sur le noyau phényle par un atome d'halogène),
leurs énantiomères, diastéréoisomères et épimères, ainsi que leurs sels d'addition à un acide ou à une base pharmaceutiquement acceptable.

2. Composés de formule (I) selon la revendication 1 dans laquelle X représente un atome d'oxygène, leurs énantiomères, diastéréoisomères et épimères, ainsi que leurs sels d'addition à un acide ou une base pharmaceutiquement acceptable.

3. Composés de formule (I) selon la revendication 1 dans laquelle X représente un groupement méthylène, leurs énantiomères, diastéréoisomères et épimères, ainsi que leurs sels d'addition à un acide ou une base pharmaceutiquement acceptable.

4. Composés de formule (I) selon la revendication 1 dans laquelle X représente un atome d'oxygène et R₁ représente un atome d'hydrogène, leurs énantiomères, diastéréoisomères et épimères, ainsi que leurs sels d'addition à un acide ou une base pharmaceutiquement acceptable.

5. Composés de formule (I) selon la revendication 1 dans laquelle R₃ et R₄ forment avec l'atome d'azote sur lequel ils sont attachés un groupement : 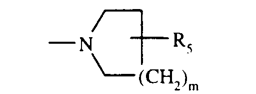 dans lequel m et R₅ sont tels que définis dans la revendication 1, leurs énantiomères, diastéréoisomères et épimères, ainsi que leurs sels d'addition à un acide ou une base pharmaceutiquement acceptable.

6. Composé de formule (I) selon la revendication 1 qui est le 5-{2-[3-(4-fluorobenzoylméthyl)pyrrolidin-1-yl]éthoxy}-1,4-benzodioxane, ses énantiomères ainsi que ses sels d'addition à un acide pharmaceutiquement acceptable.

7. Procédé de préparation des composés de formule (I) selon la revendication 1 tels que X représente un atome d'oxygène et R₁ représente un atome d'hydrogène est caractérisé en ce que l'on utilise comme produit de départ un composé de formule (II) : 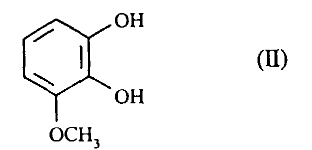 que l'on fait réagir avec du 1,2-dibromoéthane en présence de carbonate de potassium et de poudre de cuivre en milieu alcoolique,
pour conduire au composé de formule (III): 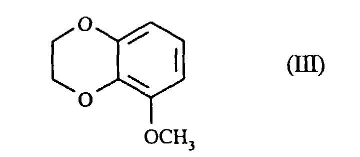 que l'on transforme, en milieu acide, en composé de formule (IV): 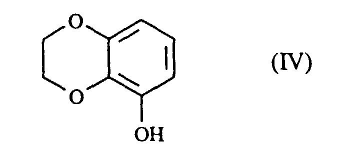 composé de formule (IV) que l'on condense:
*ou bien* sur un composé de formule (V), après réaction avec de l'éthanolate de sodium:
Br-(CH₂)ₙ-Cl (V)
dans laquelle n a la même signification que dans la formule (I),
pour conduire au composé de formule (VI) : 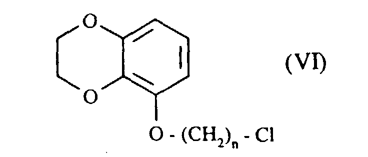 dans laquelle n a la même signification que dans la formule (I),
que l'on fait ensuite réagir sur une amine de formule (VII), dont on a éventuellement séparé les isomères : 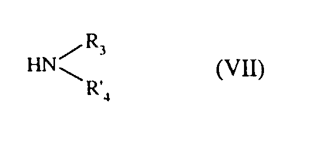 dans laquelle R'₄ a la même signification que R₄ à l'exception du cas où R₄ représente un groupement benzoyl substitué ou non,
pour conduire au composé de formule (I/a), cas particulier des composés de formule (I) : 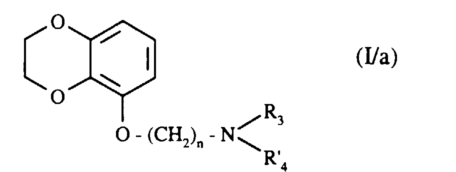 dans laquelle n, R₃ et R'₄ ont la même signification que précédemment,
*ou bien* sur un nitrile de formule (VIII), en milieu basique,
Br-(CH₂)ₙ₋₁-CN (VIII)
dans laquelle n a la même signification que dans la formule (I),
pour conduire au composé de formule (IX) : 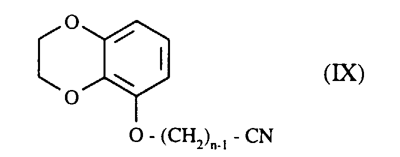 dans laquelle n a la même signification que dans la formule (I),
que l'on réduit à l'aide d'hydrure de lithium aluminium,
pour conduire au composé de formule (X) : 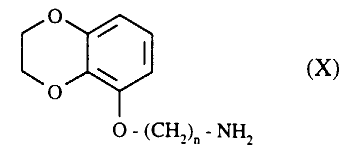 dans laquelle n a la même signification que dans la formule (I),
que l'on fait réagir sur un chlorure d'acide de formule (XI) :
Cl-CO-R''₄ (XI)
dans laquelle R''₄ représente un groupement phényl (substitué ou non par un ou plusieurs atomes d'halogène ou groupements alkyle (C₁-C₄) linéaire ou ramifié, alkoxy (C₁-C₄) linéaire ou ramifié, trihalogénométhyle ou hydroxy),
pour conduire au composé de formule (I/b), cas particulier des composés de formule (I) : 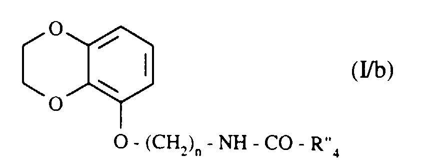 dans laquelle n et R''₄ ont la même signification que précédemment,
composé de formule (I/b) qui peut subir, si on le désire, une réduction en présence de borohydrure de tétrabutylammonium,
pour conduire au composé de formule (I/c), cas particulier des composés de formule (I) : 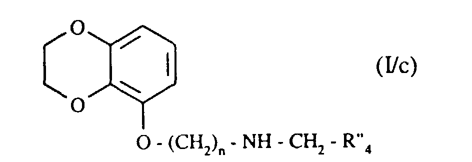 dans laquelle n et R"₄ ont la même signification que précédemment,
composé de formule (I/a), (I/b) ou (I/c):
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

8. Procédé de préparation des composés de formule (I), selon la revendication 1 tels que X représente un atome d'oxygène et R₁ représente un groupement aminocarbonyl ou hydroxyméthyle est caractérisé en ce que l'on utilise comme produit de départ un composé de formule (XII) : 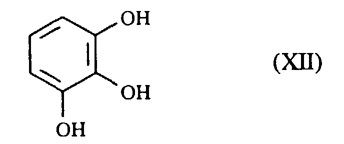 que l'on fait réagir avec le 2,3-dibromopropionate d'éthyle, en milieu basique,
pour conduire au composé de formule (XIII) : 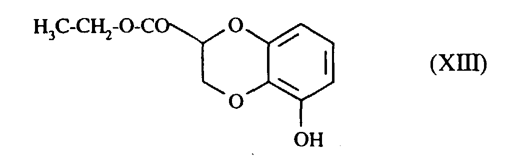 que l'on fait réagir, si on le souhaite, dans une solution ammoniacale,
pour conduire au composé de formule (XIV) : 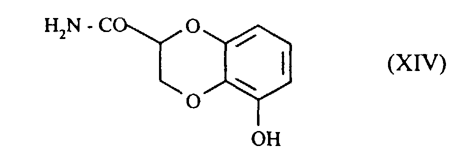 composé de formule (XIII) ou (XIV) que l'on condense :
*ou bien* sur un composé de formule (V) :
Br-(CH₂)ₙ-Cl (V)
dans laquelle n a la même signification que dans la formule (I),
pour conduire respectivement aux composés de formule (XV) ou (XVI) : 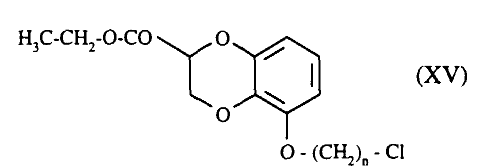 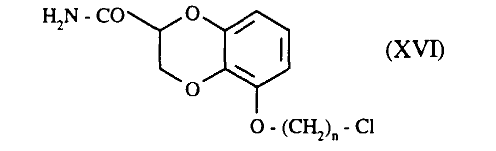 dans lesquelles n a la même signification que dans la formule (I),
que l'on fait réagir respectivement sur une amine de formule (VII), dont on a éventuellement séparé les isomères : 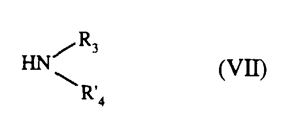 dans laquelle R'₄ a la même signification que R₄, à l'exception du cas où R₄ représente un groupement benzoyl substitué ou non,
pour conduire aux composés de formule (XVII) ou (I/d), cas particulier des composés de formule (I) : 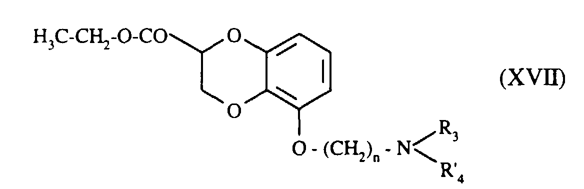 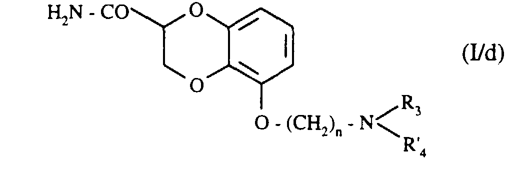 dans lesquelles n, R₃ et R'₄ ont la même signification que précédemment,
composé de formule (XVII) que l'on réduit en présence d'hydrure de lithium aluminium, pour conduire au composé de formule (I/e), cas particulier des composés de formule (I) : 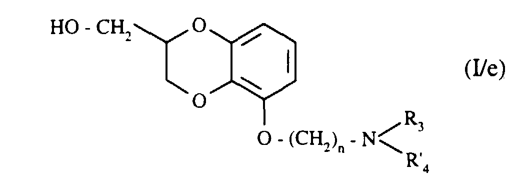 dans laquelle n, R₃ et R'₄ ont la même signification que dans la formule (I),
*ou bien*, sur un nitrile de formule (VIII), en milieu basique,
Br-(CH₂)ₙ₋₁-CN (VIII)
dans laquelle n a la même signification que dans la formule (I),
pour conduire respectivement aux composés de formule (XVIII) ou (XIX): 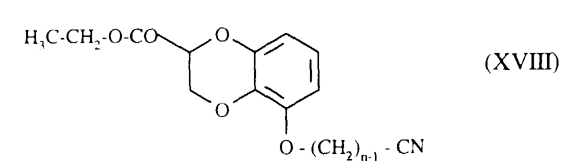 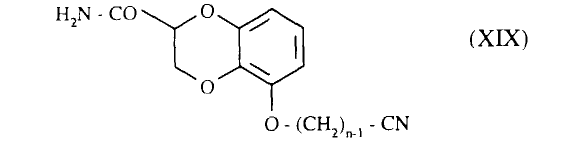 dans lesquelles n a la même signification que dans la formule (I),
que l'on réduit à l'aide d'hydrure de lithium aluminium,
pour conduire respectivement aux composés de formule (XX) ou (XXI) : 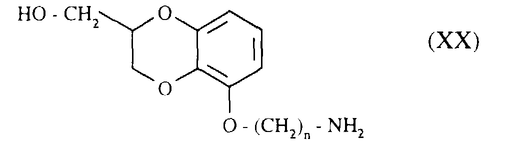 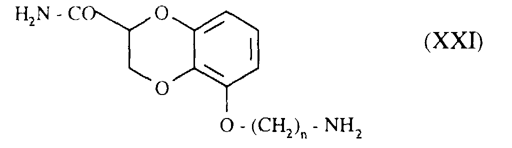 dans lesquelles n a la même signification que dans la formule (I),
que l'on fait réagir, respectivement, sur un chlorure d'acide de formule (XI) :
Cl-CO-R''₄ (XI)
dans laquelle R''₄ représente un groupement phényl (substitué ou non par un ou plusieurs atomes d'halogène ou groupements alkyle (C₁-C₄) linéaire ou ramifié, alkoxy (C₁-C₄) linéaire ou ramifié, trihalogénométhyle ou hydroxy),
pour conduire respectivement aux composés de formule (I/f) ou (I/g), cas particuliers des composés de formule (I) : 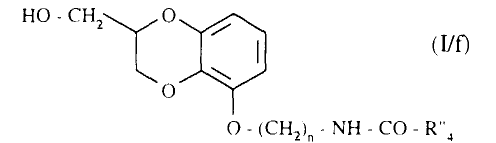 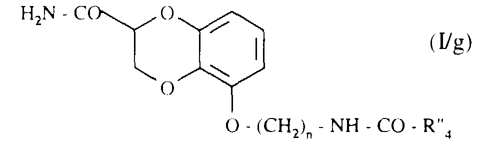 dans lesquelles n et R''₄ ont la même signification que précédemment,
composés de formule (I/f) ou (I/g), qui peuvent subir, si on le désire, une réduction en présence de borohydrure de tétrabutylammonium,
pour conduire respectivement aux composés de formule (I/h) ou (I/i), cas particuliers des composés de formule (I) : 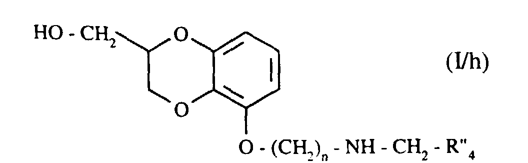 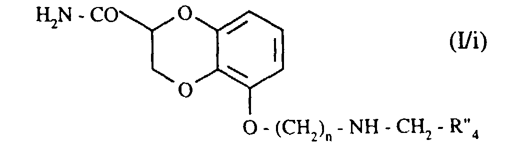 dans lesquelles n et R"₄ ont la même signification que précédemment,
composé de formule (I/d), (I/e), (I/f), (I/g), (I/h) ou (I/i):
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

9. Procédé de préparation des composés de formule (I), selon la revendication 1 tels que X représente un groupement méthylène est caractérisé en ce que l'on utilise comme produit de départ un composé de formule (XXII) : 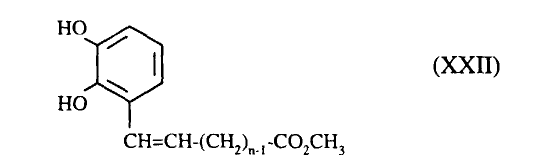 dans laquelle n a la même signification que dans la formule (I), qui subit une hydrogénation catalytique pour conduire au composé de formule (XXIII) : 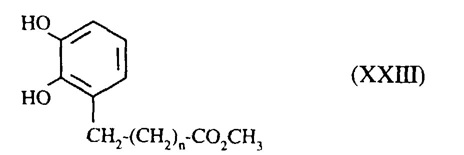 dans laquelle n a la même signification que dans la formule (I),
que l'on réduit en présence d'hydrure de lithium aluminium, pour conduire au composé de formule (XXIV): 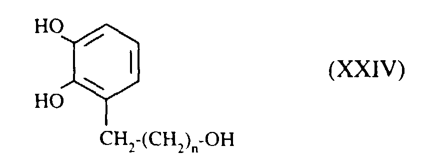 dans laquelle n a la même signification que dans la formule (I),
que l'on fait réagir, avec le 1,2-dibromoéthane ou avec le 2,3-dibromopropionate d'éthyle, selon le dérivé de formule (I), que l'on souhaite obtenir,
pour conduire au composé de formule (XXV) : 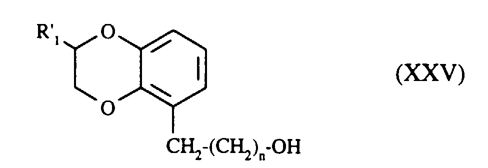 dans laquelle R'₁ représente un atome d'hydrogène ou un groupement éthoxycarbonyle,
composé de formule (XXV) (qui lorsque R'₁ représente un groupement éthoxycarbonyle peut subir, si on le souhaite, l'action d'une solution ammoniacale, pour conduire à l'amide correspondant),
que l'on met ensuite en réaction avec du tétrabromure de carbone en présence de triphénylphosphine,
pour conduire au composé de formule (XXVI), 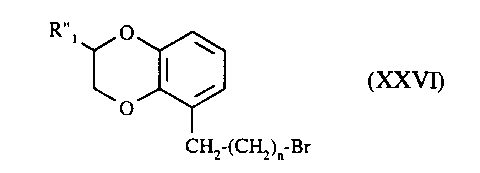 dans laquelle n a la même signification que dans la formule (I), et R''₁ représente un atome d'hydrogène, un groupement éthoxycarbonyle ou aminocarbonyle,
que l'on fait ensuite réagir avec une amine de formule (VII), dont on a éventuellement séparé les isomères : 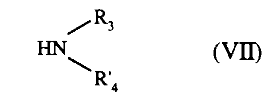 dans laquelle R'₄ a la même signification que R₄, à l'exception du cas où R₄ représente un groupement benzoyl substituté ou non,
pour conduire au composé de formule (I/j), cas particulier des composés de formule (I) (après transformation, en présence d'hydrure de lithium aluminium, du groupement R''₁ quand il représente un groupement éthoxycarbonyle, en groupement hydroxyméthyle) : 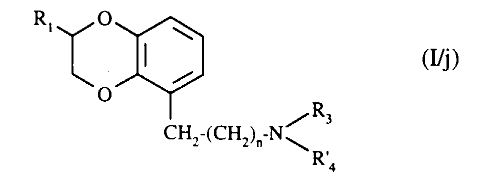 dans laquelle R₁, n, R₃ et R'₄ ont la même signification que précédemment,
composé de formule (I/j) :
- qui peut être, le cas échéant, purifié selon une technique classique de purification
- dont on sépare, le cas échéant, les isomères selon une technique classique de séparation,
- que l'on transforme, si on le souhaite, en ses sels d'addition à une base pharmaceutiquement acceptable.

10. Composition pharmaceutique contenant comme principe actif au moins un composé selon l'une quelconque des revendications 1 à 6, seul ou en combinaison avec un ou plusieurs véhicules inertes, non toxiques pharmaceutiquement acceptable.

11. Composition pharmaceutique selon la revendication 10 contenant au moins un principe actif selon l'une des revendications 1 à 6 utile dans le traitement de la schizophrénie.

## Patentansprüche

1. Verbindungen der Formel (I): 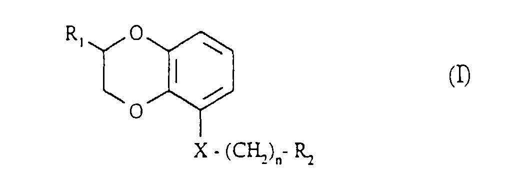 in der:
X ein Sauerstoffatom oder eine Methylgruppe darstellt,
n 1, 2 oder 3 bedeutet,
R₁ ein Wasserstoffatom, eine Aminocarbonylgruppe oder eine Hydroxymethylgruppe darstellt,
R₂ eine Gruppe: 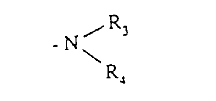 bedeutet, in der:
entweder R₃ ein Wasserstoffatom oder eine geradkettige oder verzweigte (C₁-C₆)-Alkylgruppe darstellt,
und R₄:
* eine geradkettige oder verzweigte (C₁-C₄)-Alkylgruppe darstellt,
- die nichtsubstituiert ist oder durch eine (gegebenenfalls durch ein oder mehrere Halogenatome oder geradkettige oder verzweigte (C₁-C₄)-Alkylgruppen, geradkettige oder verzweigte (C₁-C₄)-Alkoxygruppen, Trihalogenmethylgruppen oder Hydroxygruppen substituierte) Phenylgruppe substituiert ist, mit der Maßgabe, daß in diesem Fall R₁ von einem Wasserstoffatom verschieden ist,
- oder durch eine (gegebenenfalls am Phenylkern durch ein oder mehrere Halogenatome, geradkettige oder verzweigte (C₁-C₄)-Alkylgruppen, geradkettige oder verzweigte (C₁-C₄)-Alkoxygruppen, Trihalogenmethylgruppen oder Hydroxygruppen substituierte) Benzoylaminogruppe substituiert ist,
* oder eine (gegebenenfalls durch ein oder mehrere Halogenatome, geradkettige oder verzweigte (C₁-C₄)-Alkylgruppen, geradkettige oder verzweigte (C₁-C₄)-Alkoxygruppen, Trihalogenmethylgruppen oder Hydroxygruppen) substituierte Benzoylgruppe darstellt,
oder R₃ und R₄ zusammen mit dem Stickstoffatom, an das sie gebunden sind, eine Gruppe: 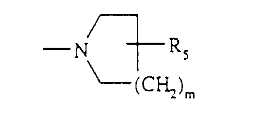 bilden, in der:
m 1, 2 oder 3 bedeutet,
R₅ eine (gegebenenfalls durch ein oder mehrere Halogenatome, geradkettige oder verzweigte (C₁-C₄)-Alkylgruppen, geradkettige oder verzweigte (C₁-C₄)-Alkoxygruppen, Hydroxygruppen oder Trihalogenmethylgruppen substituierte) Benzoylgruppe,
eine (gegebenenfalls am Phenylkern durch ein oder mehrere Halogenatome, geradkettige oder verzweigte (C₁-C₄)-Alkylgruppen, geradkettige oder verzweigte (C₁-C₄)-Alkoxygruppen, Hydroxygruppen oder Trihalogenmethylgruppen substituierte) Benzoylmethylgruppe,
eine 2-Oxo-(3H)-benzimidazol-1-yl-gruppe,
eine (gegebenenfalls am Phenylkern durch ein Halogenatom substituierte) 1H-Indazol-3-ylmethylgruppe,
oder eine (gegebenenfalls am Phenylkern durch ein Halogenatom substituierte) 1,2-Benzisoxazol-3-ylmethylgruppe darstellt,
deren Enantiomere, Diastereoisomere und Epimere sowie deren Additionssalze mit einer pharmazeutisch annehmbaren Säure oder Base.

2. Verbindungen der Formel (I) nach Anspruch 1, in der X ein Sauerstoffatom darstellt, deren Enantiomere, Diastereoisomere und Epimere sowie deren Additionssalze mit einer pharmazeutisch annehmbaren Säure oder Base.

3. Verbindungen der Formel (I) nach Anspruch 1, in der X eine Methylengruppe darstellt, deren Enantiomere, Diastereoisomere und Epimere sowie deren Additionssalze mit einer pharmazeutisch annehmbaren Säure oder Base.

4. Verbindungen der Formel (I) nach Anspruch 1, in der X ein Sauerstoffatom und R₁ ein Wasserstoffatom bedeuten, deren Enantiomere, Diastereoisomere und Epimere sowie deren Additionssalze mit einer pharmazeutisch annehmbaren Säure oder Base.

5. Verbindungen der Formel (I) nach Anspruch 1, in der R₃ und R₄ zusammen mit dem Stickstoffatom, an das sie gebunden sind, eine Gruppe: 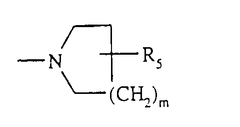 bilden, in der m und R₅ die in Anspruch 1 angegebenen Bedeutungen besitzen, deren Enantiomere, Diastereoisomere und Epimere sowie deren Additionssalze mit einer pharmazeutisch annehmbaren Säure oder Base.

6. Verbindung der Formel (I) nach Anspruch 1, nämlich 5-{2-[3-(4-Fluorbenzoylmethyl)-pyrrolidin-1-yl)-ethoxy}-1,4-benzodioxan, dessen Enantiomere sowie dessen Additionssalze mit einer pharmazeutisch annehmbaren Säure.

7. Verfahren zur Herstellung der Verbindungen der Formel (I) nach Anspruch 1, worin X ein Sauerstoffatom und R₁ ein Wasserstoffatom bedeuten, dadurch gekennzeichnet, daß man als Ausgangsprodukt eine Verbindung der Formel (II) verwendet: 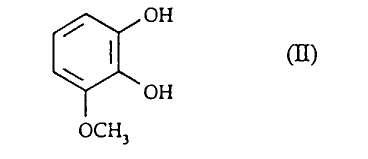 welche man mit 1,2-Dibromethan in Gegenwart von Kaliumcarbonat und Kupferpulver in alkoholischem Medium umsetzt,
zur Bildung der Verbindung der Formel (III): 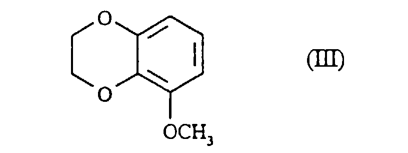 welche man in saurem Medium in die Verbindung der Formel (IV) umwandelt: 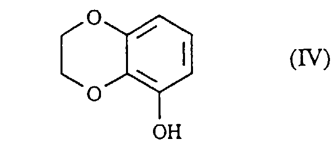 welche Verbindung der Formel (IV) man:
*entweder* nach der Umsetzung mit Natriumethanolat mit einer Verbindung der Formel (V):
Br-(CH₂)ₙ-Cl (V)
kondensiert, in der n die bezüglich der Formel (I) angegebenen Bedeutungen besitzt, zur Bildung der Verbindung der Formel (VI): 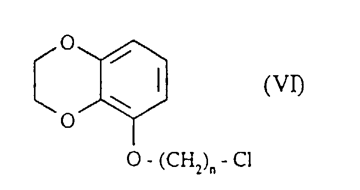 in der n die bezüglich der Formel (I) angegebenen Bedeutungen besitzt,
welche man anschließend mit einem Amin der Formel (VII), welches man gegebenenfalls in die Isomeren aufgetrennt hat: 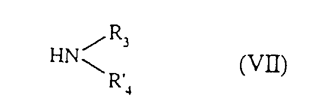 in der R'₄ die gleichen Bedeutungen besitzt wie R₄ mit Ausnahme des Falls, wo R₄ eine gegebenenfalls substituierte Benzoylgruppe darstellt, umsetzt,
zur Bildung der Verbindung der Formel (I/a), einem Sonderfall der Verbindungen der Formel (I): 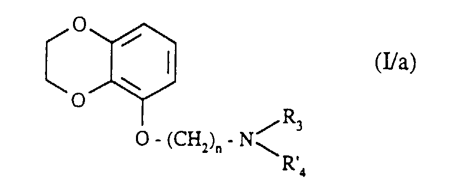 in der n, R₃ und R'₄ die oben angegebenen Bedeutungen besitzen,
*oder* in basischem Medium mit einem Nitril der Formel (VIII):
Br-(CH₂)ₙ₋₁-CN (VIII)
kondensiert, in der n die bezüglich der Formel (I) angegebenen Bedeutungen besitzt,
zur Bildung der Verbindung der Formel (IX): 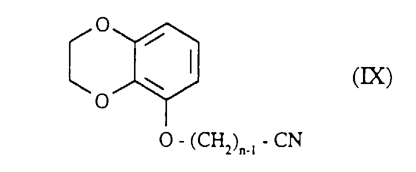 in der n die bezüglich der Formel (I) angegebenen Bedeutungen besitzt, welche man mit Lithiumaluminiumhydrid reduziert,
zur Bildung der Verbindung der Formel (X): 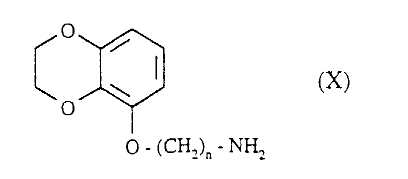 in der n die bezüglich der Formel (I) angegebenen Bedeutungen besitzt, welche man mit einem Säurechlorid der Formel (XI):
Cl-CO-R"₄ (XI)
in der R"₄ eine (gegebenenfalls durch ein oder mehrere Halogenatome, geradkettige oder verzweigte (C₁-C₄)-Alkylgruppen, geradkettige oder verzweigte (C₁-C₄)-Alkoxygruppen, Trihalogenmethylgruppen oder Hydroxygruppen substituierte) Phenylgruppe darstellt, umsetzt,
zur Bildung der Verbindung der Formel (I/b), einem Sonderfall der Verbindungen der Formel (I): 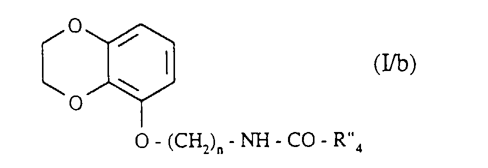 in der n und R"₄ die oben angegebenen Bedeutungen besitzen,
welche Verbindung der Formel (I/b) man gewünschtenfalls einer Reduktion in Gegenwart von Tetrabutylammoniumborhydrid unterwerfen kann,
zur Bildung der Verbindung der Formel (I/c), einem Sonderfall der Verbindungen der Formel (I): 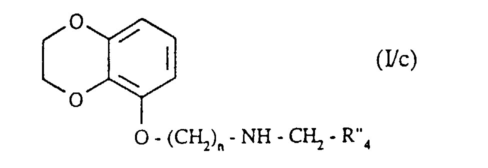 in der n und R"₄ die oben angegebenen Bedeutungen besitzen,
welche Verbindungen der Formeln (I/a), (I/b) und (I/c) man:
- gegebenenfalls mit Hilfe einer klassischen Reinigungsmethode reinigen kann,
- gegebenenfalls mit Hilfe einer klassischen Trennmethode in die Isomeren trennen kann,
- gewünschtenfalls mit einer pharmazeutisch annehmbaren Base in ihre Additionssalze umwandeln kann.

8. Verfahren zur Herstellung der Verbindungen der Formel (I) nach Anspruch 1, worin X ein Sauerstoffatom und R₁ eine Aminocarbonyl- oder Hydroxymethylgruppe bedeuten, dadurch gekennzeichnet, daß man als Ausgangsprodukt eine Verbindung der Formel (XII) verwendet: 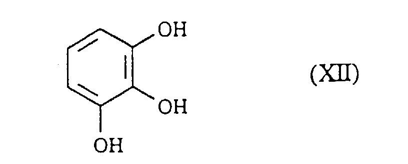 welche man mit 2,3-Dibrompropionsäureethylester in basischem Medium umsetzt,
zur Bildung der Verbindung der Formel (XIII): 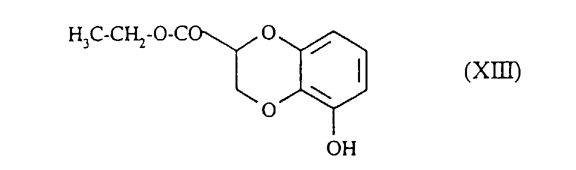 welche man gewünschtenfalls in einer ammoniakalischen Lösung umsetzt,
zur Bildung der Verbindung der Formel (XIV): 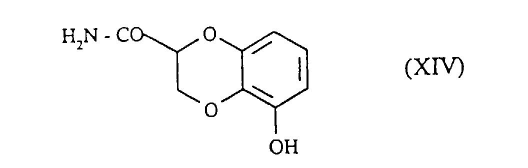 welche Verbindung der Formel (XIII) oder (XIV) man:
*entweder* mit einer Verbindung der Formel (V):
Br - (CH₂)ₙ - Cl (V)
in der n die bezüglich der Formel (I) angegebenen Bedeutungen besitzt, kondensiert,
zur Bildung der Verbindungen der Formel (XV) bzw. (XVI): 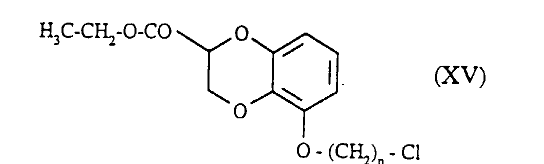 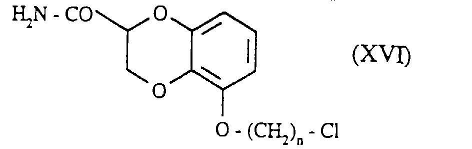 in denen n die bezüglich der Formel (I) angegebenen Bedeutungen besitzt,
welche man jeweils mit einem Amin der Formel (VII), welches man gegebenenfalls in die Isomeren aufgetrennt hat: 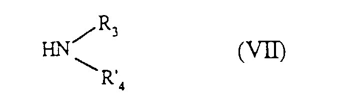 in der R'₄ die Bedeutungen von R₄ besitzt mit Ausnahme des Falls, da R₄ eine gegebenenfalls substituierte Benzoylgruppe darstellt, umsetzt,
zur Bildung der Verbindungen der Formel (XVII) oder (I/d), einem Sonderfall der Verbindungen der Formel (I): 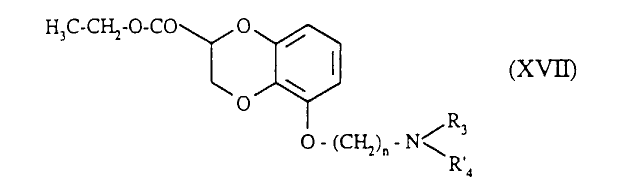 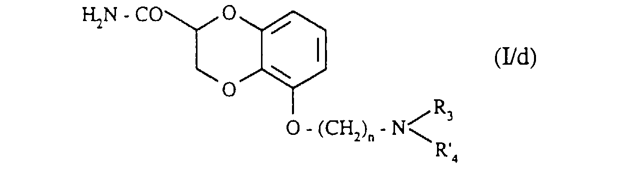 worin n, R₃ und R'₄ die oben angegebenen Bedeutungen besitzen,
worauf man die Verbindung der Formel (XVII) in Gegenwart von Lithiumaluminiumhydrid reduziert,
zur Bildung der Verbindungen der Formel (I/e), einem Sonderfall der Verbindungen der Formel (I): 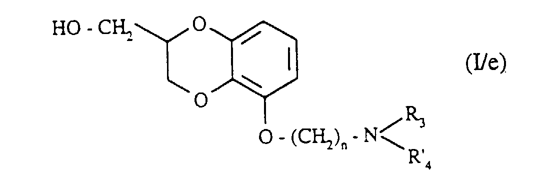 in der n, R₃ und R'₄ die bezüglich der Formel (I) angegebenen Bedeutungen besitzen,
*oder* in basischem Medium mit einem Nitril der Formel (VIII):
Br - (CH₂)ₙ₋₁ - CN (VIII)
in der n die bezüglich der Formel (I) angegebenen Bedeutungen besitzt, kondensiert,
zur Bildung der Verbindungen der Formel (XVIII) bzw. (XIX): 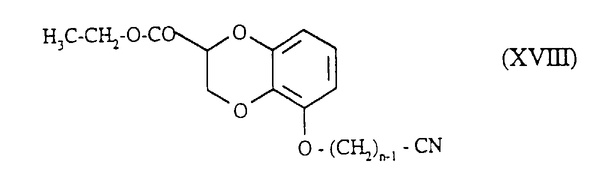 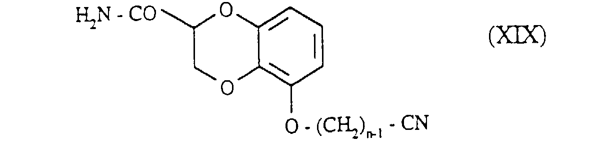 worin n die bezüglich der Formel (I) angegebenen Bedeutungen besitzt, welche man mit Hilfe von Lithiumaluminiumhydrid reduziert,
zur Bildung der Verbindungen der Formel (XX) bzw. (XXI): 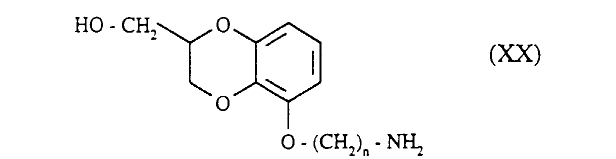 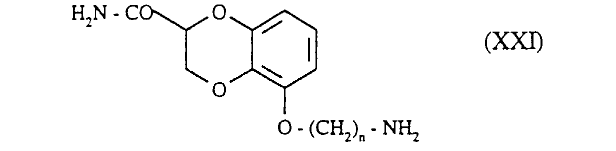 worin n die bezüglich der Formel (I) angegebenen Bedeutungen besitzt,
welche man jeweils mit einem Säurechlorid der Formel (XI) umsetzt:
Cl - CO - R"₄ (XI)
in der R"₄ eine (gegebenenfalls durch ein oder mehrere Halogenatome, geradkettige oder verzweigte (C₁-C₄)-Alkylgruppen, geradkettige oder verzweigte (C₁-C₄)-Alkoxygruppen, Trihalogenmethylgruppen oder Hydroxygruppen substituierte) Phenylgruppe darstellt,
zur Bildung der Verbindungen der Formel (I/f) bzw. (I/g), Sonderfällen der Verbindungen der Formel (I): 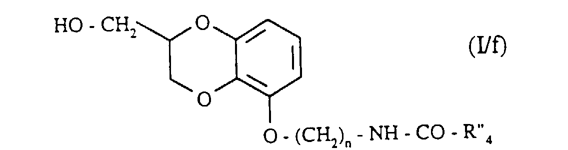 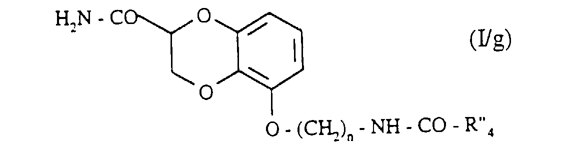 worin n und R"₄ die oben angegebenen Bedeutungen besitzen,
welche Verbindungen der Formel (I/f) oder (I/g) gewünschtenfalls einer Reduktion in Gegenwart von Tetrabutylammoniumborhydrid unterworfen werden können, zur Bildung der Verbindungen der Formel (I/h) bzw. (I/i), Sonderfällen der Verbindungen der Formel (I): 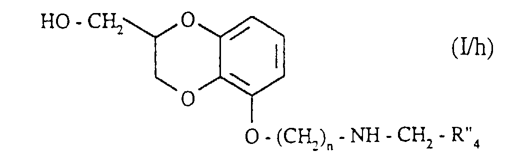 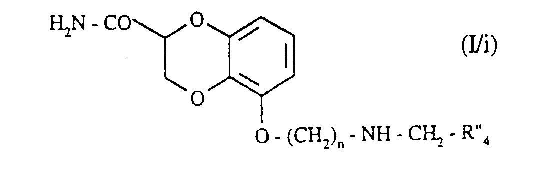 worin n und R"₄ die oben angegebenen Bedeutungen besitzen,
welche Verbindungen der Formeln (I/d), (I/e), (I/f), (I/g), (I/h) und (I/i) man:
- gegebenenfalls mit Hilfe einer klassischen Reinigungsmethode reinigen kann,
- gegebenenfalls mit Hilfe einer klassischen Trennmethode in die Isomeren trennen kann,
- gewünschtenfalls mit einer pharmazeutisch annehmbaren Base in ihre Additionssalze umwandeln kann.

9. Verfahren zur Herstellung der Verbindungen der Formel (I) nach Anspruch 1, worin X eine Methylengruppe darstellt, dadurch gekennzeichnet, daß man als Ausgangsprodukt eine Verbindung der Formel (XXII) verwendet: 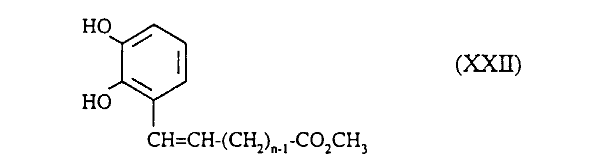 in der n die bezüglich der Formel (I) angegebenen Bedeutungen besitzt, welche man einer katalytischen Hydrierung unterwirft zur Bildung der Verbindung der Formel (XXIII): 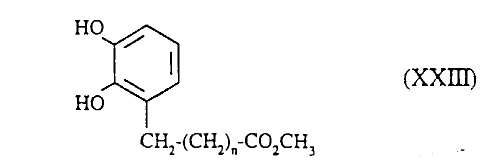 in der n die bezüglich der Formel (I) angegebenen Bedeutungen besitzt,
welche man in Gegenwart von Lithiumaluminiumhydrid reduziert zur Bildung der Verbindung der Formel (XXIV): 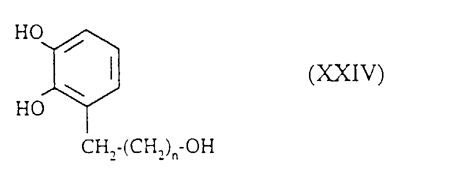 in der n die bezüglich der formel (I) angegebenen Bedeutungen besitzt,
welche man in Abhängigkeit von dem herzustellenden Derivat der Formel (I) mit 1,2-Dibromethan oder mit 2,3-Dibrompropionsäureethylester umsetzt,
zur Bildung der Verbindung der Formel XXV): 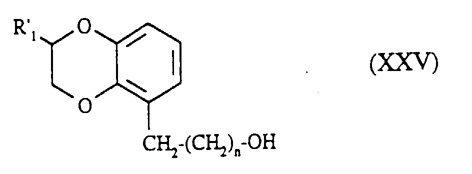 in der R'₁ ein Wasserstoffatom oder eine Ethoxycarbonylgruppe darstellt,
welche Verbindung der Formel (XXV) (welche, wenn R'₁ eine Ethoxycarbonylgruppe darstellt, gewünschtenfalls der Einwirkung einer Ammoniaklösung unterworfen werden kann zur Bildung des entsprechenden Amids),
man anschließend mit Kohlenstofftetrabromid in Gegenwart von Triphenylphosphin umsetzt,
zur Bildung der Verbindung der Formel (XXVI): 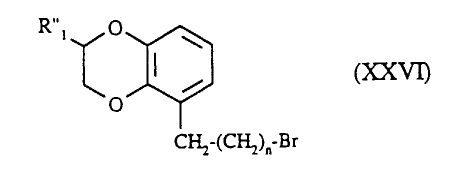 in der n die bezüglich der Formel (I) angegebenen Bedeutungen besitzt und R"₁ ein Wasserstoffatom, eine Ethoxycarbonylgruppe oder eine Aminocarbonylgruppe bedeutet,
welche man anschließend mit einem Amin der Formel (VII), welches man eventuell in die Isomeren aufgespalten hat: 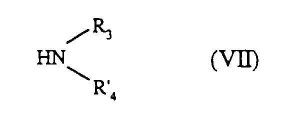 in der R'₄ die gleichen Bedeutungen besitzt wie R₄ mit Ausnahme des Falls, da R₄ eine gegebenenfalls substituierte Benzoylgruppe darstellt, umsetzt,
zur Bildung der Verbindung der Formel (I/j), einem Sonderfall der Verbindungen der Formel (I) (nach der Umwandlung der Gruppe R"₁, wenn es sich dabei um eine Ethoxycarbonylgruppe handelt, in Gegenwart von Lithiumaluminiumhydrid in eine Hydroxymethylgruppe): 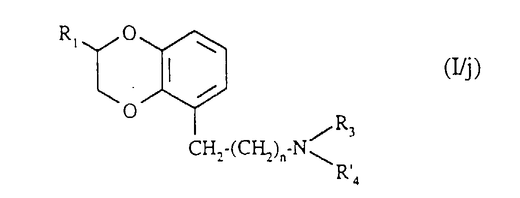 in der R₁, n, R₃ und R'₄ die oben angegebenen Bedeutungen besitzen, welche Verbindung der Formel (I/j) man:
- gegebenenfalls mit Hilfe einer klassischen Reinigungsmethode reinigen kann,
- gegebenenfalls mit Hilfe einer klassischen Trennmethode in die Isomeren trennen kann,
- gewünschtenfalls mit einer pharmazeutisch annehmbaren Base in ihre Additionssalze umwandeln kann.

10. Pharmazeutische Zubereitung, enthaltend als Wirkstoff mindestens eine Verbindung nach einem der Ansprüche 1 bis 6, allein oder in Kombination mit einem oder mehreren inerten, nichttoxischen, pharmazeutisch annehmbaren Trägermaterialien.

11. Pharmazeutische Zubereitung nach Anspruch 10, enthaltend mindestens einen Wirkstoff nach einem der Ansprüche 1 bis 6 zur Behandlung der Schizophrenie.

## Claims

1. Compounds of formula (I): 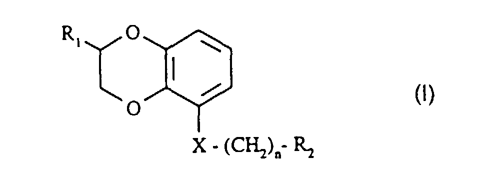 in which:
X represents an oxygen atom or a methylene group,
n represents 1, 2 or 3,
R₁ represents a hydrogen atom, an aminocarbonyl group or a hydroxymethyl group,
R₂ represents a group: 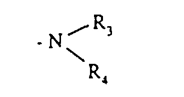 in which:
either R₃ represents a hydrogen atom or a linear or branched (C₁-C₆)alkyl group,
and R₄ represents:
* a linear or branched (C₁-C₄)alkyl group which is
- unsubstituted or substituted by a phenyl group (which is unsubstituted or substituted by one or more halogen atoms or linear or branched (C₁-C₄)alkyl, linear or branched (C₁-C₄)alkoxy, trihalomethyl or hydroxy groups), with the proviso that in those cases, R₁ is other than a hydrogen atom,
- or substituted by a benzoylamino group (which is unsubstituted or substituted on the phenyl nucleus by one or more halogen atoms or linear or branched (C₁-C₄)alkyl, linear or branched (C₁-C₄)alkoxy, trihalomethyl or hydroxy groups),
* or a benzoyl group (which is unsubstituted or substituted by one or more halogen atoms or linear or branched (C₁-C₄)alkyl, linear or branched (C₁-C₄)alkoxy, trihalomethyl or hydroxy groups),
or R₃ and R₄, with the nitrogen atom to which they are bonded, form a group: 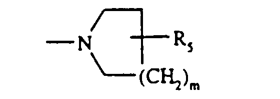 in which:
m represents 1, 2 or 3,
R₅ represents a benzoyl group (which is unsubstituted or substituted by one or more halogen atoms or linear or branched (C₁-C₄)alkyl, linear or branched (C₁-C₄)alkoxy, hydroxy or trihalomethyl groups),
a benzoylmethyl group (which is unsubstituted or substituted on the phenyl nucleus by one or more halogen atoms or linear or branched (C₁-C₄)alkyl, linear or branched (C₁-C₄)alkoxy, hydroxy or trihalomethyl groups),
a 2-oxo-(3H)-benzimidazol-1-yl group,
a 1H-indazol-3-ylmethyl group (which is unsubstituted or substituted on the phenyl nucleus by a halogen atom),
or a 1,2-benzisoxazol-3-ylmethyl group (which is unsubstituted or substituted on the phenyl nucleus by a halogen atom),
their enantiomers, diastereoisomers and epimers, and addition salts thereof with a pharmaceutically acceptable acid or base.

2. Compounds of formula (I) according to claim 1 in which X represents an oxygen atom, their enantiomers, diastereoisomers and epimers, and addition salts thereof with a pharmaceutically acceptable acid or base.

3. Compounds of formula (I) according to claim 1 in which X represents a methylene group, their enantiomers, diastereoisomers and epimers, and addition salts thereof with a pharmaceutically acceptable acid or base.

4. Compounds of formula (I) according to claim 1 in which X represents an oxygen atom and R₁ represents a hydrogen atom, their enantiomers, diastereoisomers and epimers, and addition salts thereof with a pharmaceutically acceptable acid or base.

5. Compounds of formula (I) according to claim 1 in which R₃ and R₄, with the nitrogen atom to which they are bonded, form a group: 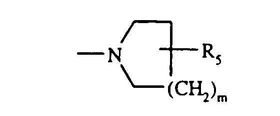 in which m and R₅ are as defined in claim 1, their enantiomers, diastereoisomers and epimers, and addition salts thereof with a pharmaceutically acceptable acid or base.

6. Compound of formula (I) according to claim 1 which is 5-{2-[3-(4-fluorobenzoylmethyl)pyrrolidin-1-yl]ethoxy}-1,4-benzodioxane, its enantiomers and its addition salts with a pharmaceutically acceptable acid.

7. Process for the preparation of compounds of formula (I) according to claim 1 wherein X represents an oxygen atom and R₁ represents a hydrogen atom, characterised in that there is used as starting material a compound of formula (II): 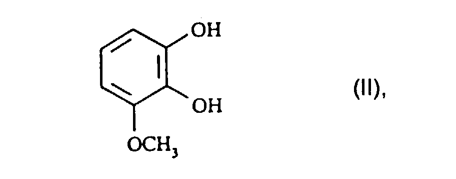 which is reacted with 1,2-dibromoethane in the presence of potassium carbonate and of copper powder in an alcoholic medium,
to yield the compound of formula (III): 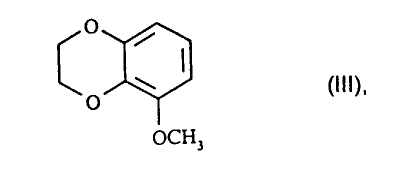 which is converted, in an acid medium, into the compound of formula (IV): 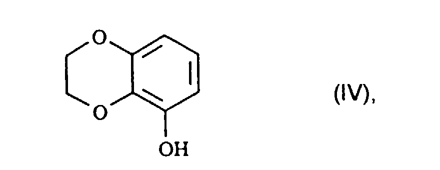 which compound of formula (IV) is condensed:
*either,* after reaction with sodium ethanolate, with a compound of formula (V):
Br - (CH₂)ₙ - Cl (V),
in which n is as defined in formula (I),
to yield a compound of formula (VI): 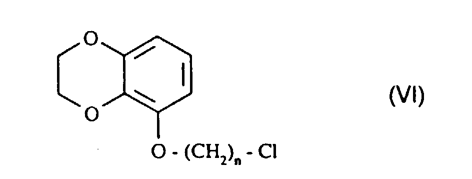 in which n is as defined in formula (I),
which is then reacted with an amine of formula (VII), which has optionally been separated into its isomers: 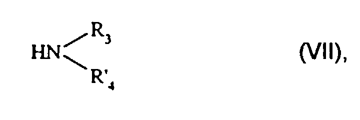 in which R'₄ has the same meaning as R₄ with the exception of the case in which R₄ represents an unsubstituted or substituted benzoyl group,
to yield a compound of formula (I/a), a particular case of the compounds of formula (I): 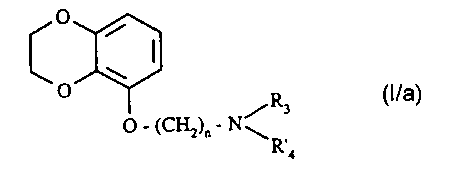 in which n, R₃ and R'₄ are as defined above,
*or* with a nitrile of formula (VIII), in a basic medium,
Br - (CH₂)ₙ₋₁ - CN (VIII),
in which n is as defined in formula (I),
to yield a compound of formula (IX): 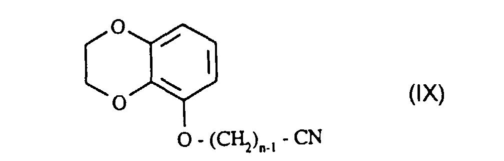 in which n is as defined in formula (I),
which is reduced by means of lithium aluminium hydride to yield a compound of formula (X): 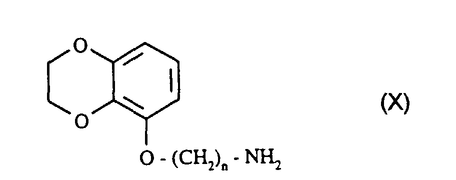 in which n is as defined in formula (I),
which is reacted with an acid chloride of formula (XI):
Cl - CO - R"₄ (XI),
in which R"₄ represents a phenyl group (which is unsubstituted or substituted by one or more halogen atoms or linear or branched (C₁-C₄)alkyl, linear or branched (C₁-C₄)alkoxy, trihalomethyl or hydroxy groups),
to yield a compound of formula (I/b), a particular case of the compounds of formula (I): 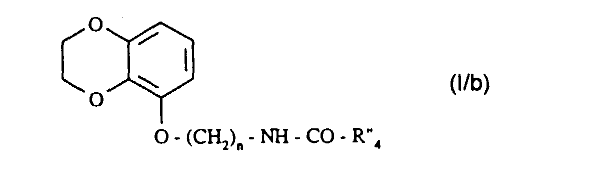 in which n and R"₄ are as defined above,
which compound of formula (I/b) may, if desired, undergo reduction in the presence of tetrabutylammonium borohydride
to yield a compound of formula (I/c), a particular case of the compounds of formula (I): 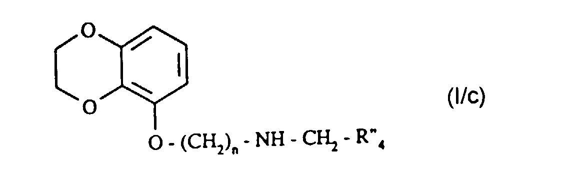 in which n and R"₄ are as defined above,
which compound of formula (I/a), (I/b) or (I/c):
- may, where appropriate, be purified by a conventional purification technique,
- is separated, where appropriate, into its isomers by a conventional separation technique,
- is converted, if desired, into its addition salts with a pharmaceutically acceptable base.

8. Process for the preparation of compounds of formula (I) according to claim 1 wherein X represents an oxygen atom and R₁ represents an aminocarbonyl or hydroxymethyl group, characterised in that there is used as starting material a compound of formula (XII): 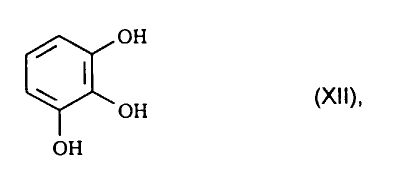 which is reacted with ethyl 2,3-dibromopropionate, in a basic medium, to yield the compound of formula (XIII): 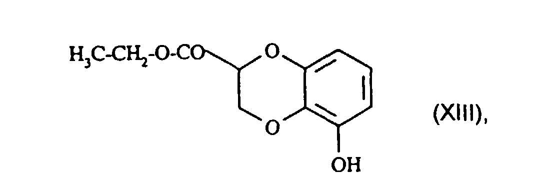 which is reacted, if desired, in an ammoniacal solution,
to yield the compound of formula (XIV): 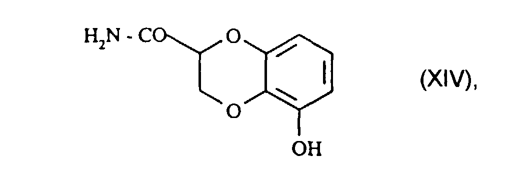 which compound of formula (XIII) or (XIV) is condensed:
*either* with a compound of formula (V):
Br - (CH₂)ₙ - Cl (V),
in which n is as defined in formula (I),
to yield a compound of formula (XV) or (XVI), respectively: 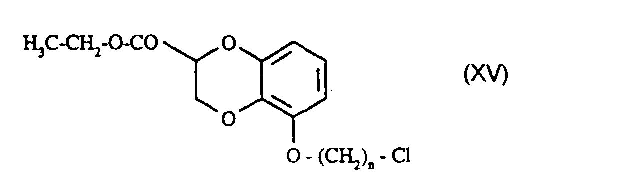 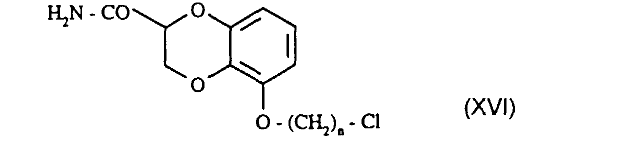 in which n is as defined in formula (I),
each of which is reacted with an amine of formula (VII), which has optionally been separated into its isomers: 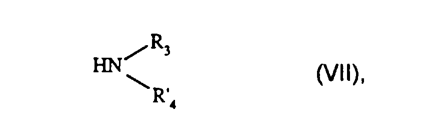 in which R'₄ has the same meaning as R₄ with the exception of the case in which R₄ represents an unsubstituted or substituted benzoyl group,
to yield a compound of formula (XVII) or (I/d), a particular case of the compounds of formula (I): 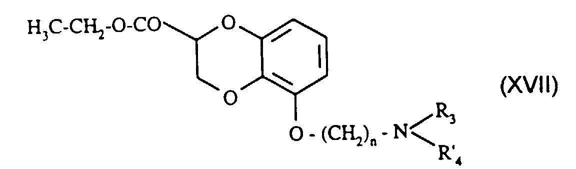 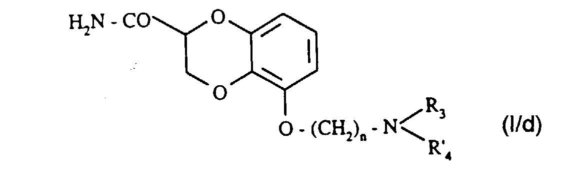 in which n, R₃ and R'₄ are as defined above,
which compound of formula (XVII) is reduced in the presence of lithium aluminium hydride
to yield a compound of formula (I/e), a particular case of the compounds of formula (I): 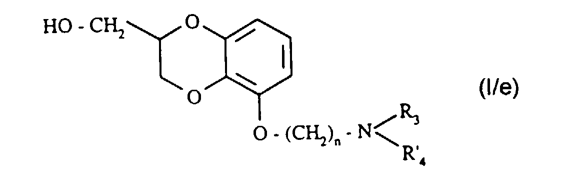 in which n, R₃ and R'₄ are as defined in formula (I),
*or* with a nitrile of formula (VIII), in a basic medium,
Br - (CH₂)ₙ₋₁ - CN (VIII),
in which n is as defined in formula (I),
to yield a compound of formula (XVIII) or (XIX), respectively: 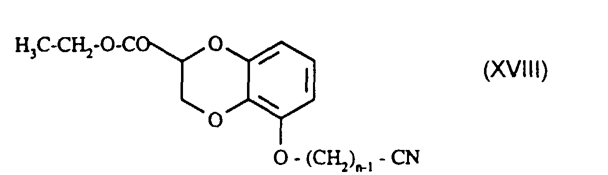 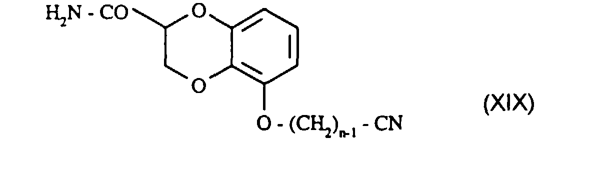 in which n is as defined in formula (I),
each of which is reduced by means of lithium aluminium hydride to yield a compound of formula (XX) or (XXI), respectively: 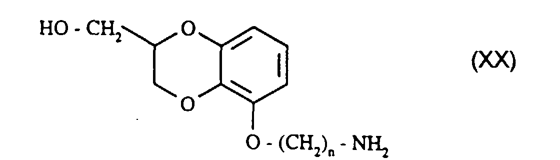 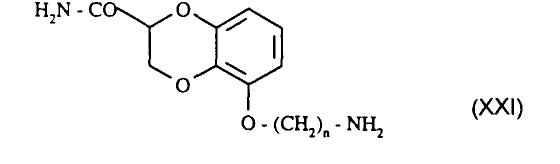 in which n is as defined in formula (I),
each of which is reacted with an acid chloride of formula (XI):
Cl - CO - R"₄ (XI),
in which R"₄ represents a phenyl group (which is unsubstituted or substituted by one or more halogen atoms or linear or branched (C₁-C₄)alkyl, linear or branched (C₁-C₄)alkoxy, trihalomethyl or hydroxy groups),
to yield a compound of formula (I/f) or (I/g), respectively, particular cases of the compounds of formula (I): 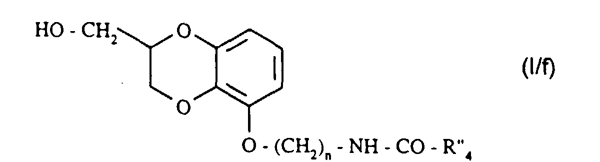 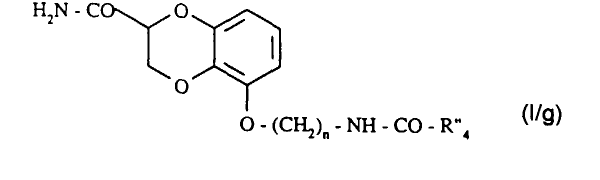 in which n and R"₄ are as defined above,
which compound of formula (I/f) or (I/g) may, if desired, undergo reduction in the presence of tetrabutylammonium borohydride
to yield a compound of formula (I/h) or (I/i), respectively, particular cases of the compounds of formula (I): 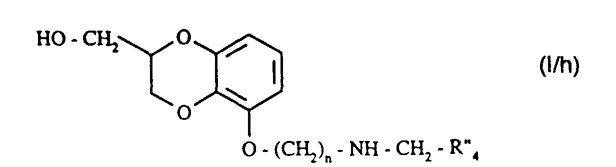 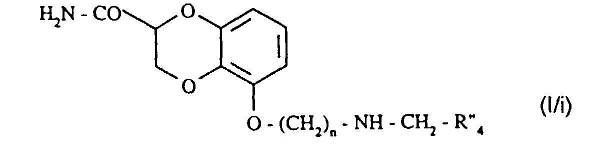 in which n and R"₄ are as defined above,
which compound of formula (I/d), (I/e), (I/f), (I/g), (I/h) or (I/i):
- may, where appropriate, be purified by a conventional purification technique,
- is separated, where appropriate, into its isomers by a conventional separation technique,
- is converted, if desired, into its addition salts with a pharmaceutically acceptable base.

9. Process for the preparation of compounds of formula (I) according to claim 1 wherein X represents a methylene group, characterised in that there is used as starting material a compound of formula (XXII): 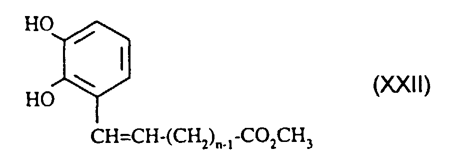 in which n is as defined in formula (I), which is subjected to catalytic hydrogenation
to yield a compound of formula (XXIII): 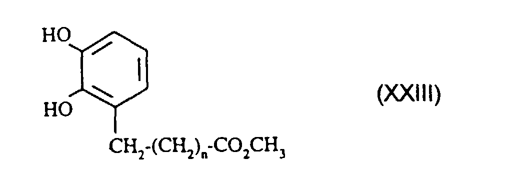 in which n is as defined in formula (I),
which is reduced in the presence of lithium aluminium hydride to yield a compound of formula (XXIV): 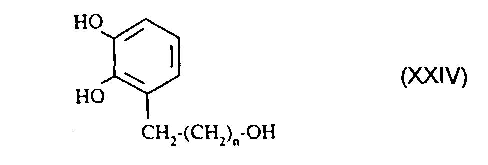 in which n is as defined in formula (I),
which is reacted with 1,2-dibromoethane or with ethyl 2,3-dibromopropionate according to the compound of formula (I) which is to be obtained,
to yield a compound of formula (XXV): 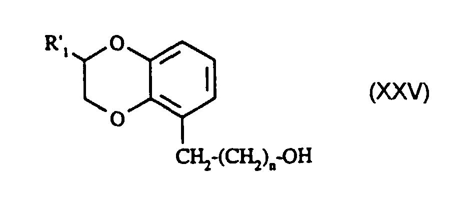 in which R'₁ represents a hydrogen atom or an ethoxycarbonyl group,
which compound of formula (XXV) (which, when R'₁ represents an ethoxycarbonyl group, may, if desired, be subjected to the action of an ammoniacal solution to yield the corresponding amide) is then reacted with carbon tetrabromide in the presence of triphenylphosphine
to yield a compound of formula (XXVI): 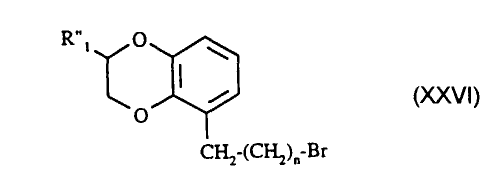 in which n is as defined in formula (I) and R"₁ represents a hydrogen atom, an ethoxycarbonyl group or an aminocarbonyl group,
which is then reacted with an amine of formula (VII), which has optionally been separated into its isomers: 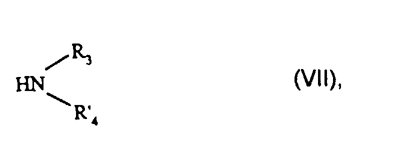 in which R'₄ has the same meaning as R₄ with the exception of the case in which R₄ represents an unsubstituted or substituted benzoyl group,
to yield a compound of formula (I/j), a particular case of the compounds of formula (I) (after conversion, in the presence of lithium aluminium hydride, of the R"₁ group, when it represents an ethoxycarbonyl group, into a hydroxymethyl group): 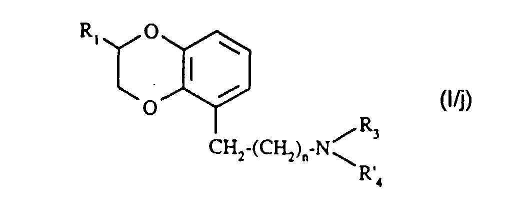 in which R₁, n, R₃ and R'₄ are as defined above,
which compound of formula (I/j):
- may, where appropriate, be purified by a conventional purification technique,
- is separated, where appropriate, into its isomers by a conventional separation technique,
- is converted, if desired, into its addition salts with a pharmaceutically acceptable base.

10. Pharmaceutical composition comprising as active ingredient at least one compound according to any one of claims 1 to 6, alone or in combination with one or more pharmaceutically acceptable inert, non-toxic carriers.

11. Pharmaceutical composition according to claim 10 comprising at least one active ingredient according to any one of claims 1 to 6, for use in the treatment of schizophrenia.
